# EUROPEAN PATENT APPLICATION

(11) **EP 1 826 207 A1**
(43) Date of publication of application: **29.08.2007**
(21) Application number: 05806810.7
(22) Date of filing: 14.11.2005
(51) Int. Cl.: C07D 409/06, A61K 31/4184, A61P 9/00, A61P 9/02, A61P 11/00, A61P 13/12, A61P 17/02, A61P 19/08, A61P 29/00, A61P 37/08, A61P 43/00

(54) **ACID SALT OF BENZIMIDAZOLE DERIVATIVE AND CRYSTAL THEREOF**

(30) Priority: 12.11.2004 JP 2004328843
(71) Applicant: Teijin Pharma Limited, Tokyo 100-0011 (JP)
(72) Inventor: TERAMOTO, Mitsuru, Teijin Pharma Ltd., Iwakuni-shi, Yamaguchi, 740-0014 (JP); TSUCHIYA, Naoki, Teijin Pharma Ltd., Hino-shi, Tokyo, 191-0065 (JP); SAITOH, Hiroshi, Teijin Pharma Ltd., Hino-shi, Tokyo, 191-0065 (JP)
(74) Representative: Hallybone, Huw George
(86) International application number: PCT/JP2005/021196
(87) International publication number: WO 2006/052026

(57) **Abstract**

The present invention provides acid salts, such as sulfate, hydrochloride, and methanesulfonate, of 4-(1-((4-methylbenzothiophen-3-yl)methyl)benzimidazol-2-ylthio)butanoic acid and the crystals thereof. These benzimidazole derivatives and the crystals thereof have an in vivo chymase inhibitory activity and can be used as a preventive or therapeutic agent for inflammatory diseases, allergic diseases, respiratory diseases, circulatory diseases, or bone/cartilage metabolic diseases.

## Description

### Technical Field

The present invention relates to an acid salt of 4-(1-((4-methylbenzothiophen-3-yl)methyl)benzimidazol-2-ylthio)butanoic acid, a crystal thereof, and a pharmaceutical composition comprising the salt or crystal. More particularly, the present invention relates to an acid salt, such as sulfate, hydrochloride, and methanesulfonate, of 4-(1-((4-methylbenzothiophen-3-yl)methyl)benzimidazol-2-ylthio)butanoic acid that has an in vivo chymase inhibitory activity and can be used as a preventive or therapeutic agent for inflammatory diseases, allergic diseases, respiratory diseases, circulatory diseases or bone/cartilage metabolic diseases; a crystal thereof; and a pharmaceutical composition comprising the same.

### Background Art

When a compound forms two or more crystal states, these different crystalline states are called crystal polymorphs. It is commonly known that the stability sometimes differs among different crystal forms of a polymorphic substance. For example, Patent Document 1 describes that two crystal forms of prazosin hydrochloride differ in stability and that the difference affects the results of long-term storage stability. Patent Document 2 describes that a particular crystal form is advantageous among various crystal forms of buspirone hydrochloride in terms of retention of particular physical properties under storage and manufacturing conditions.

In manufacturing of a drug substance for pharmaceuticals, it is generally advantageous to obtain a drug substance as crystals in terms of storage stability of the drug substance and pharmaceutical compositions, control of the manufacturing processes, and others.

When a compound that has two or more crystal forms is utilized for a pharmaceutical, the different crystal forms differ in physicochemical properties such as melting point, solubility, stability, and others and in pharmacokinetics (absorption, distribution, metabolism, excretion, etc.); as a result, they may have different biological properties such as pharmacological efficacy. In order to guarantee that these properties of a pharmaceutical are constant, it is often required to manufacture a drug substance in a particular crystal form. Also, in the process of manufacturing a drug substance, it is sometimes important to produce a particular crystal form in crystallization to keep the yield and purification effect constant.

It is impossible to anticipate the presence of crystal polymorphs by the structure of compound and it is regarded important to find crystal polymorphs in the development of pharmaceuticals.

It is known that 4-(1-((4-methylbenzothiophen-3-yl)methyl)benzimidazol-2-ylthio)butanoic acid represented by formula (I) below has an inhibitory effect on chymase as described in Patent Document 3, Patent Document 4, and Patent Document 5.

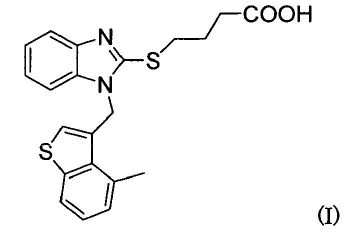

As pharmaceutically acceptable non-toxic salts, Patent Document 3 and Patent Document 4 describe carboxylate salts with a metal ion such as Na⁺, ammonium ion, or the like as a counter cation; however, neither these documents nor Patent Document 5 describes anything about salts with anionic counter ion such as sulfate. As a matter of course, there is no description on crystals or crystal polymorphism of acid salts.

Chymase is one of neutral proteases present in mast cell granules and has an important role in various biological responses in which mast cells are involved. There have been reported various effects, for example, promotion of degranulation from mast cell, activation of interleukin-1β (IL-1β), activation of matrix protease, degradation of fibronectin and collagen type IV, promotion of release of transforming growth factor-β (TGF-β), activation of substance P and vasoactive intestinal polypeptide (VIP), conversion from angiotensin (Ang) I to AngII, and conversion of endothelin. Based on the above findings, inhibitors against the activities of chymase are considered to be promising as preventive and/or therapeutic agents for respiratory diseases such as bronchial asthma; inflammatory/allergic diseases such as allergic rhinitis, atopic dermatitis, and urticaria; circulatory diseases such as sclerotic vascular lesion, intravascular stenosis, peripheral circulatory disturbance, renal insufficiency, and cardiac insufficiency; bone/cartilage metabolic diseases such as rheumatoid and osteoarthritis; or the like.
Patent Document 1: Japanese Patent Laid-open Publication No. S62-226980
Patent Document 2: Japanese Patent Laid-open Publication No. S64-71816
Patent Document 3: WO International Publication No. 00/03997 pamphlet
Patent Document 4: WO International Publication No. 01/53291 pamphlet
Patent Document 5: WO International Publication No. 2004/101551 pamphlet

### Disclosure of the Invention

An object of the present invention is to provide an acid salt, such as sulfate, hydrochloride, or methanesulfonate, of 4-(1-((4-methylbenzothiophen-3-yl)methyl)benzimidazol-2-ylthio)butanoic acid and a crystal thereof.

Another object of the present invention is to provide a preventive and/or therapeutic agent that has a chymase inhibitory activity for inflammatory diseases, allergic diseases, respiratory diseases, circulatory diseases, or bone/cartilage metabolic diseases.

The present inventors have, as a result of their intensive studies, found that 4-(1-((4-methylbenzothiophen-3-yl)methyl)benzimidazol-2-ylthio)butanoic acid forms acid salts such as sulfate, hydrochloride, and methanesulfonate, and the sulfate crystallizing in five crystal forms, the hydrochloride crystallizing in three crystal forms, the methanesulfonate crystallizing in five crystal forms, and that all of these crystals are suitable as a drug substance for a pharmaceutical composition or a manufacturing intermediate thereof, and they have completed the present invention.

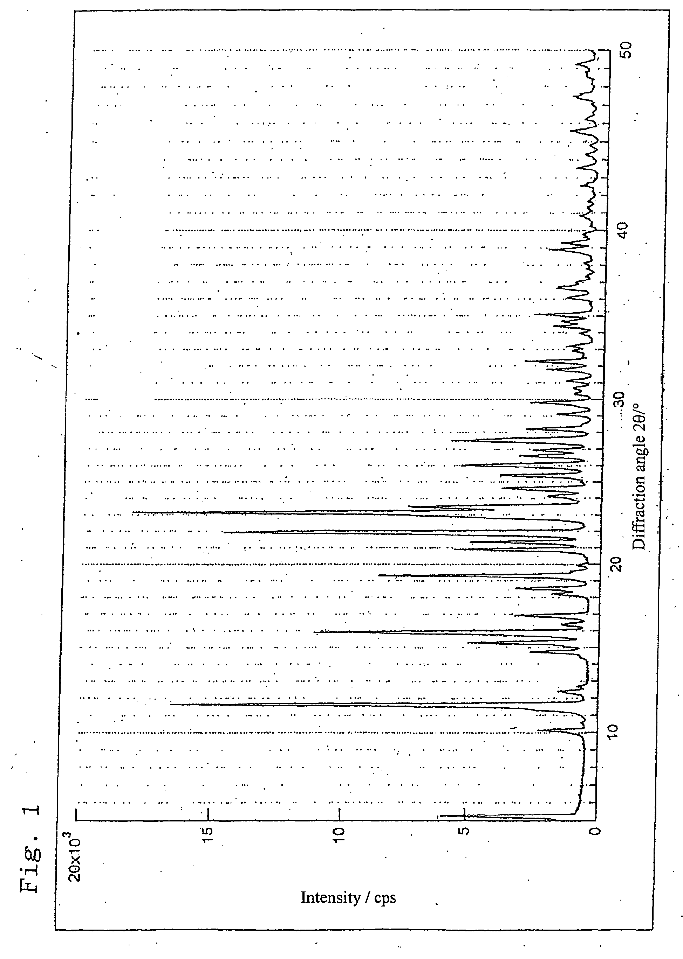

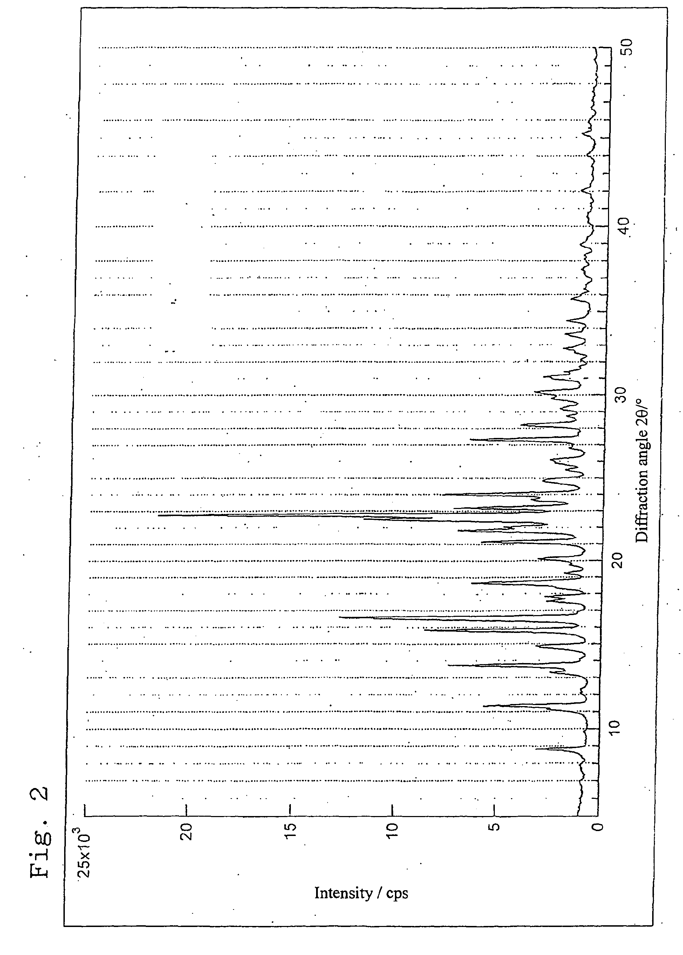

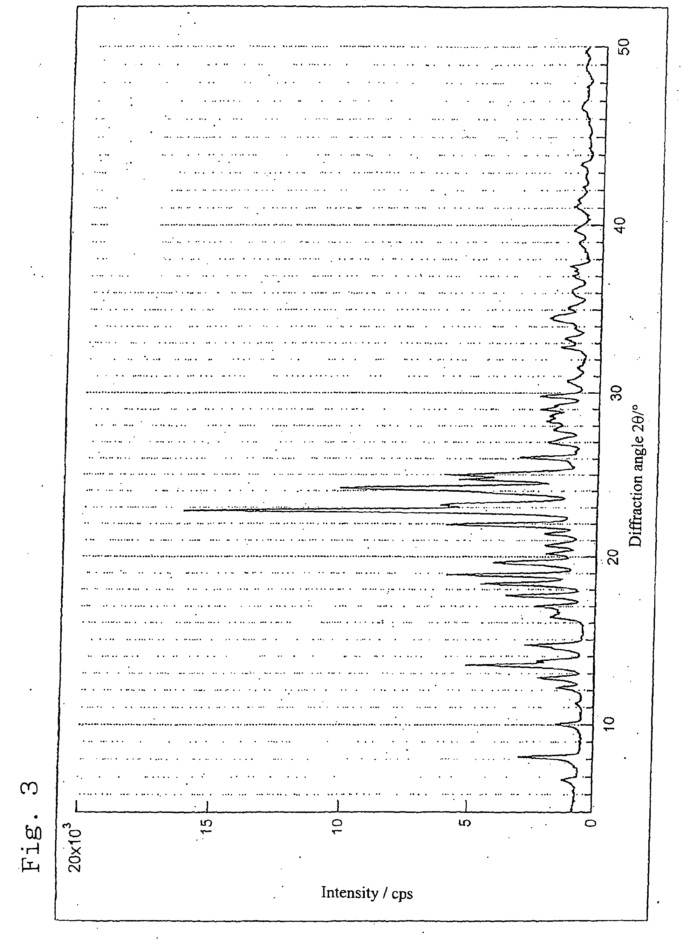

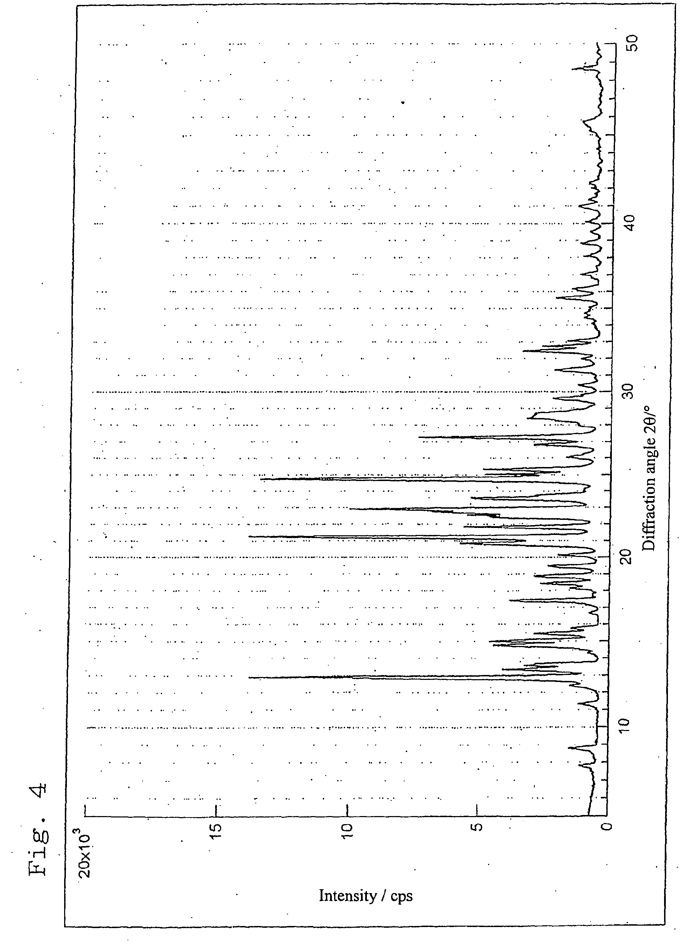

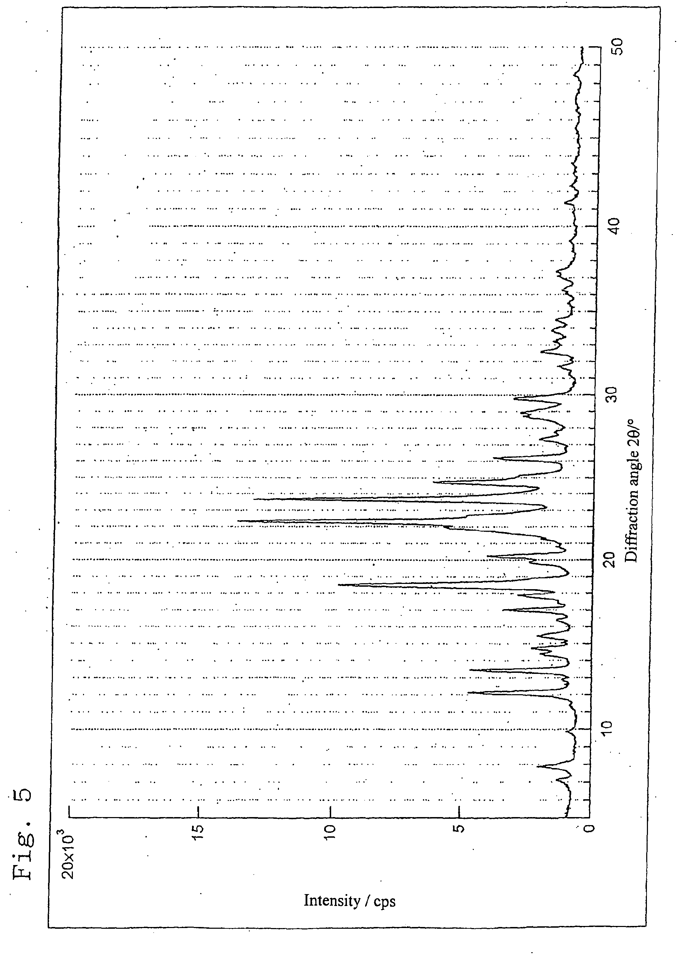

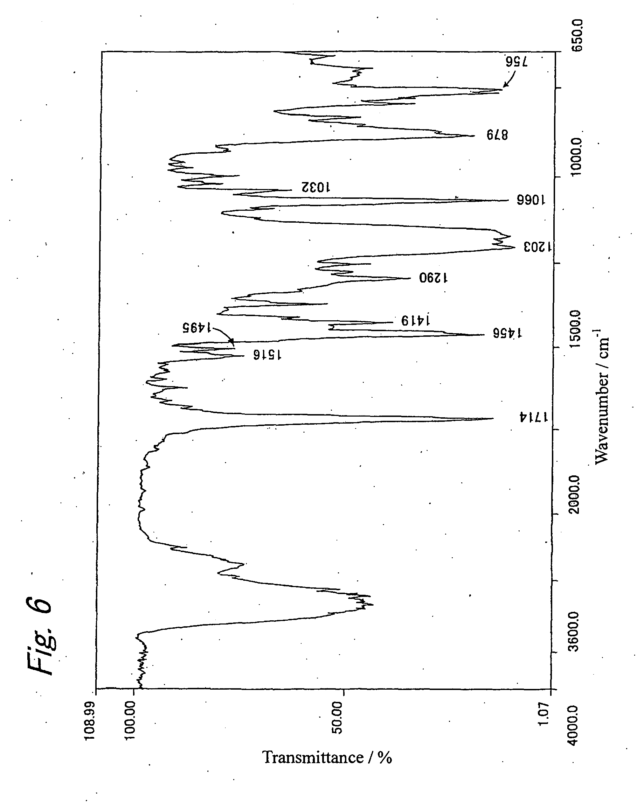

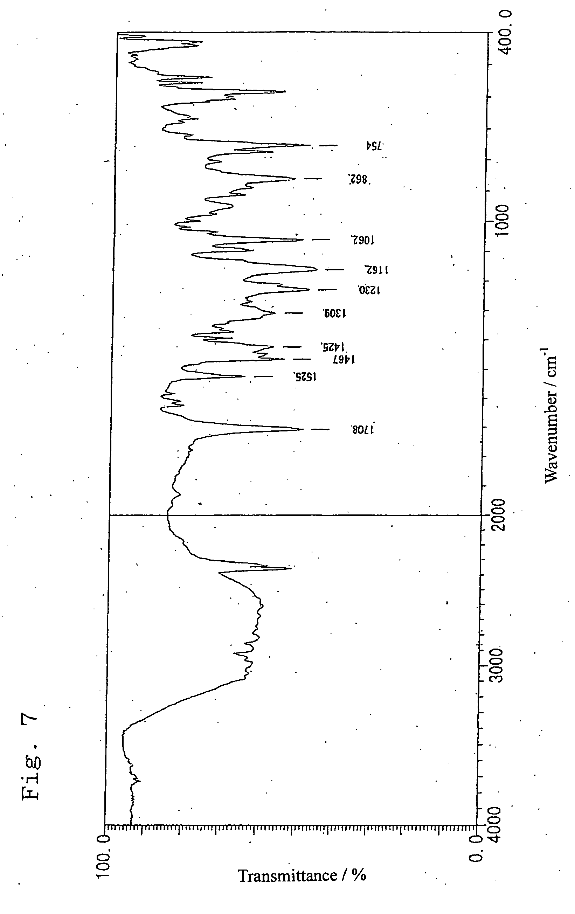

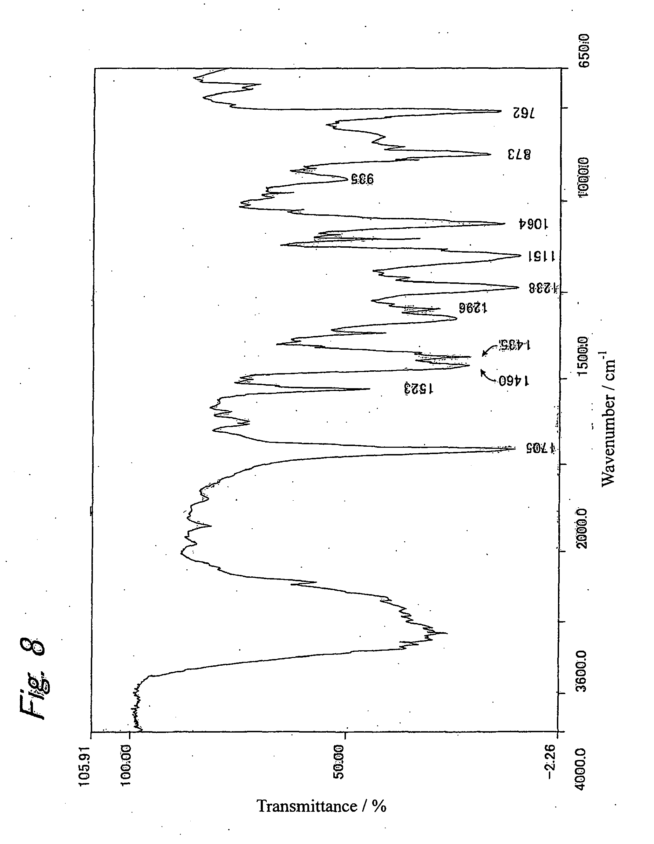

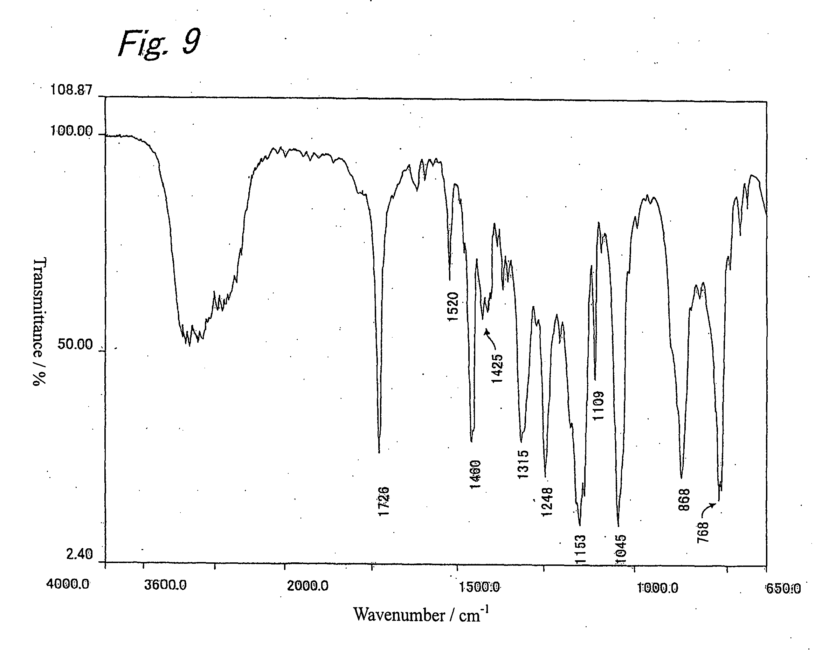

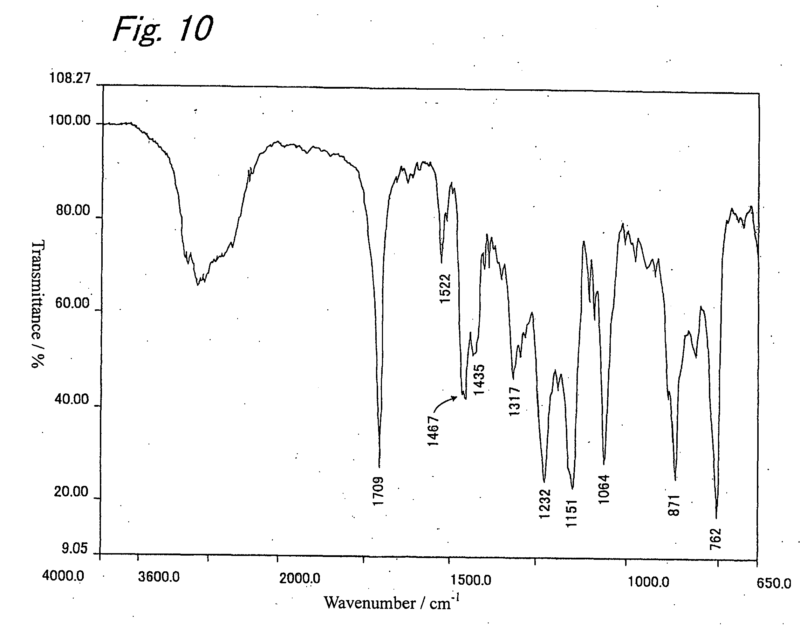

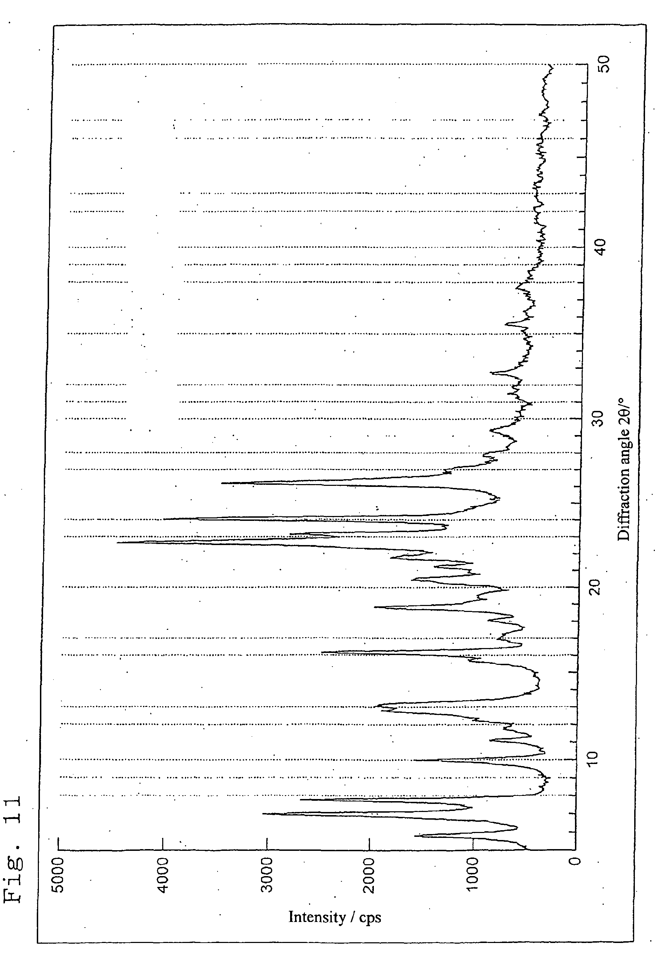

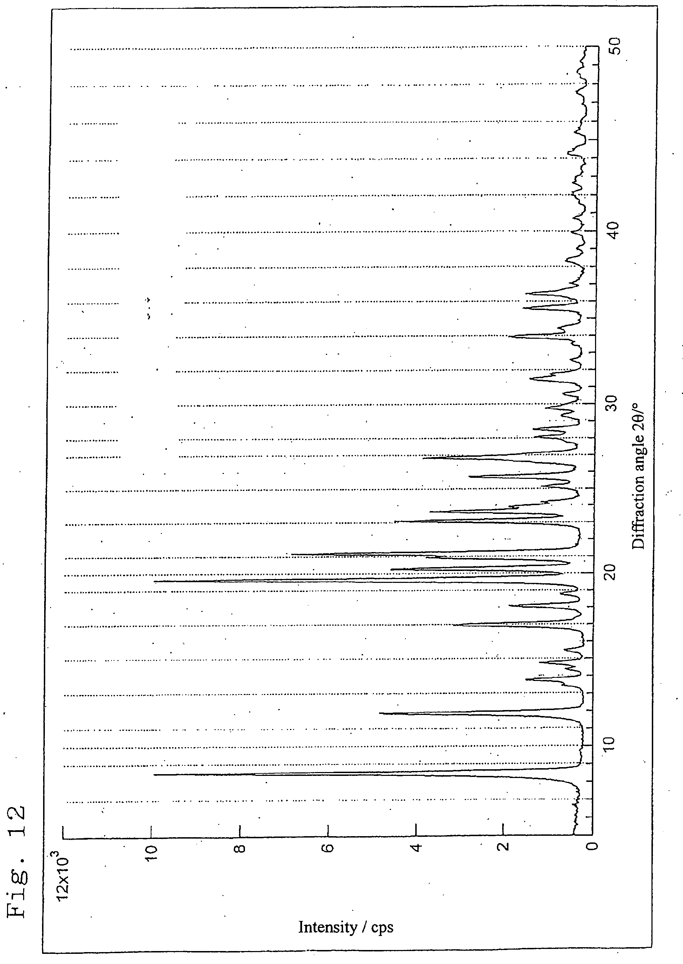

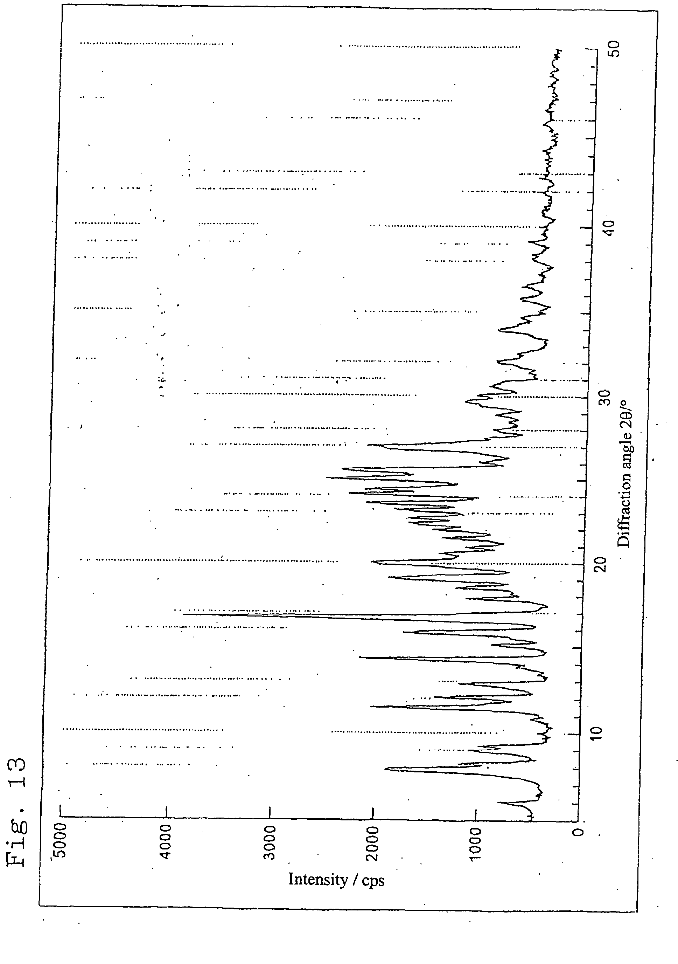

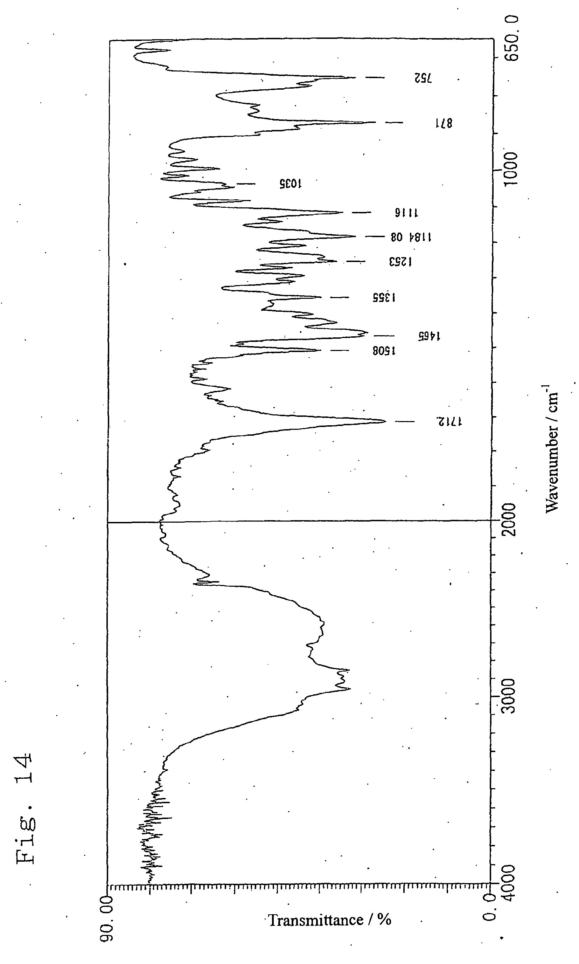

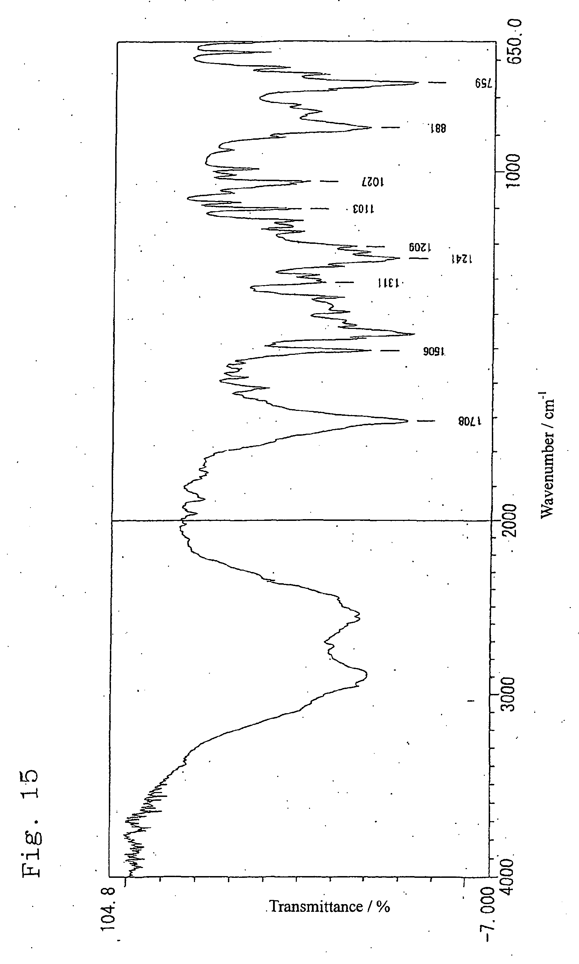

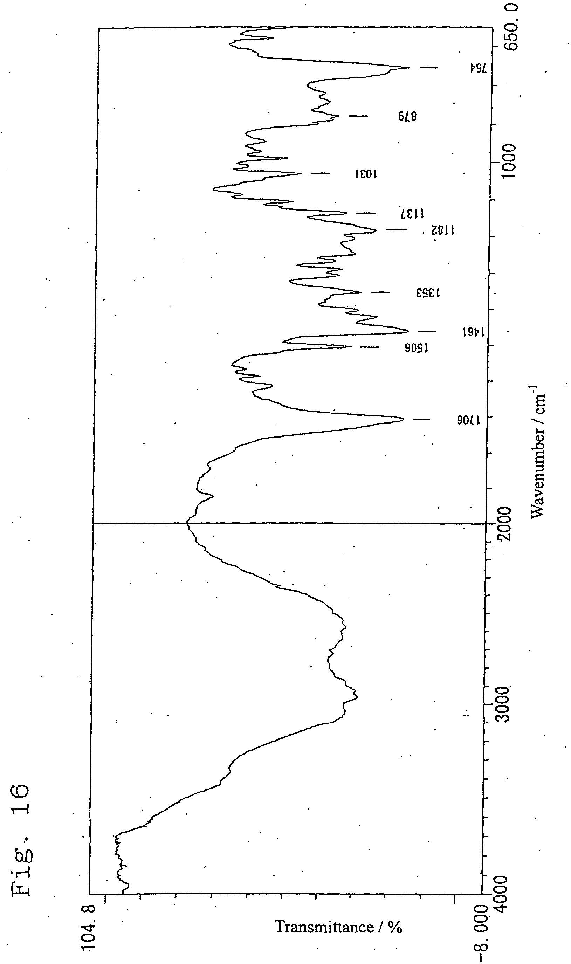

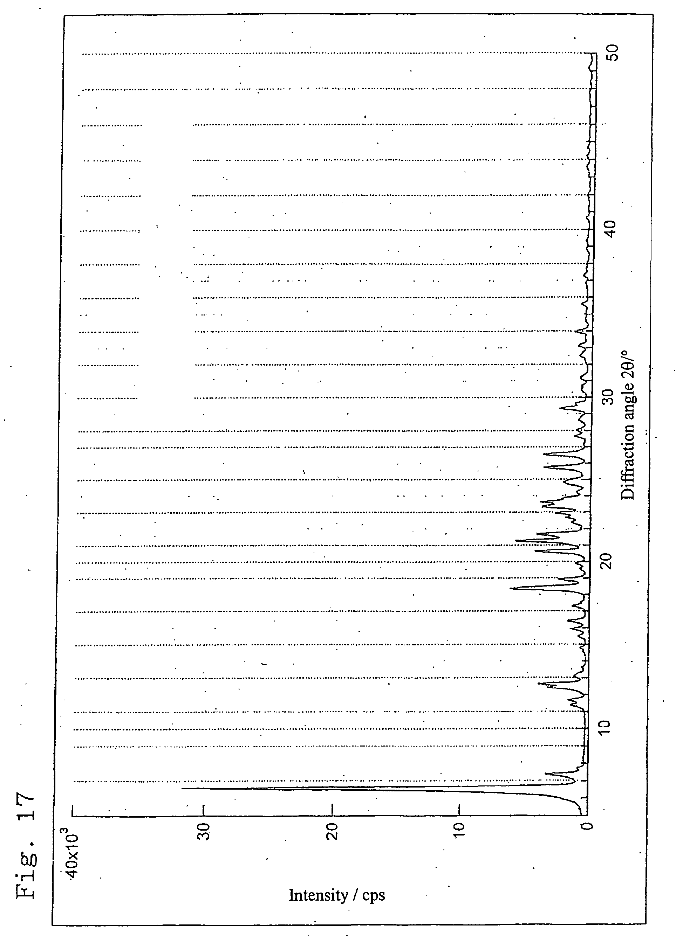

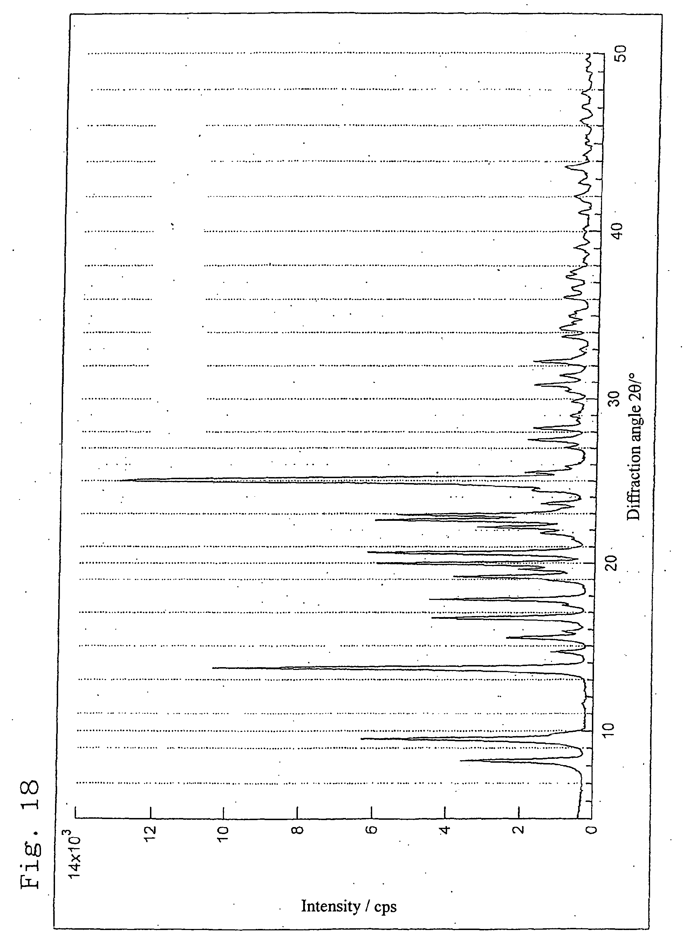

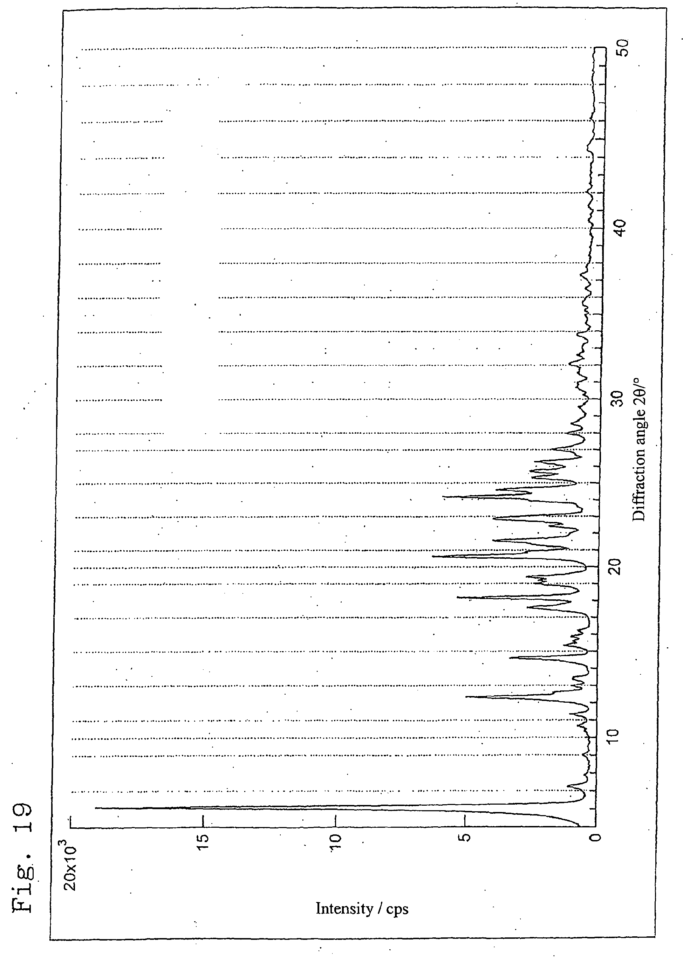

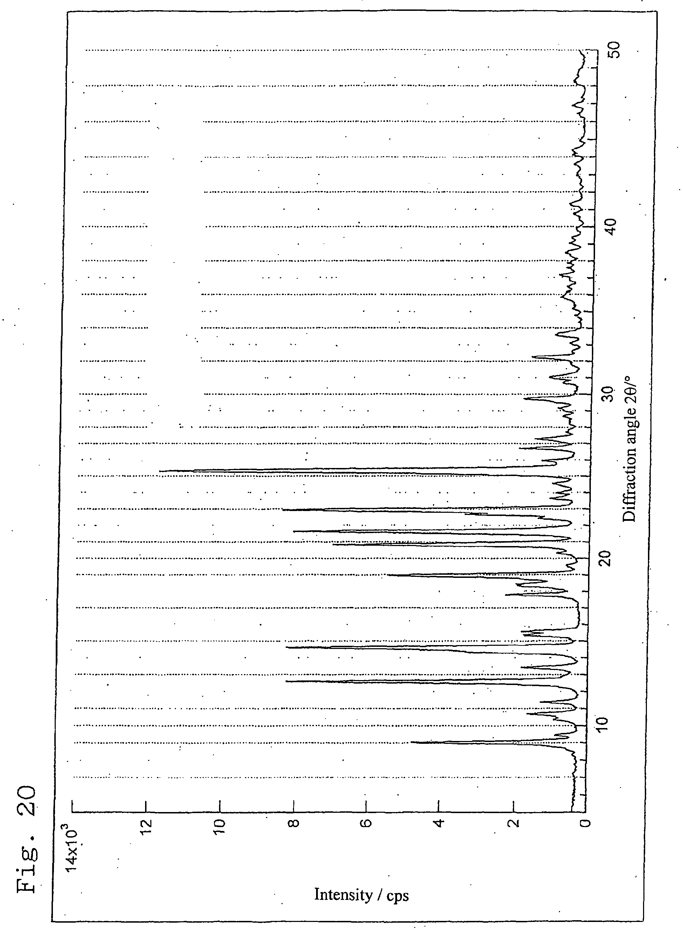

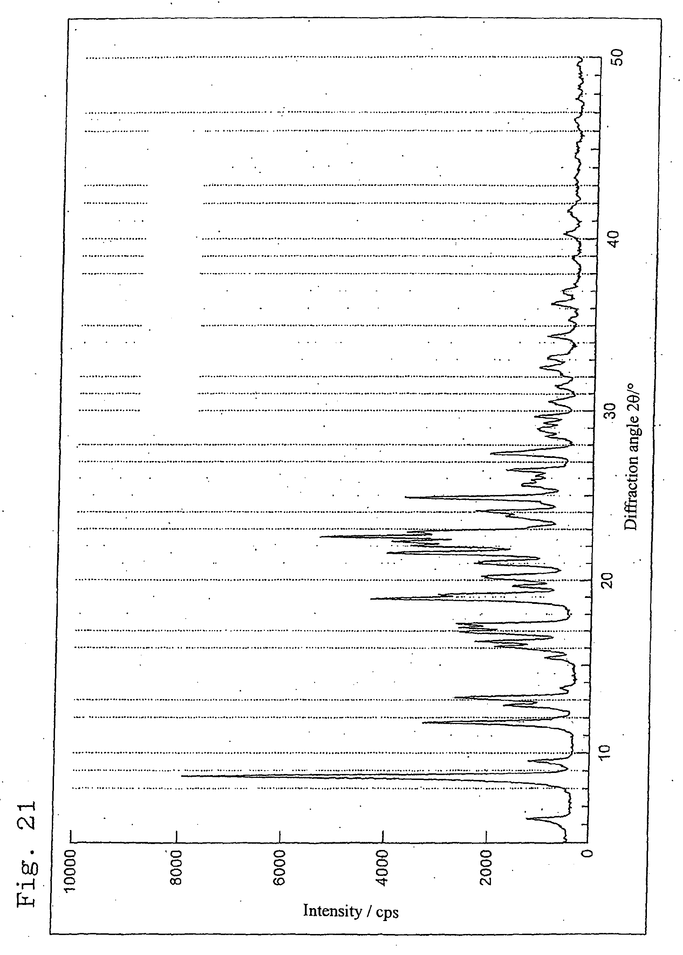

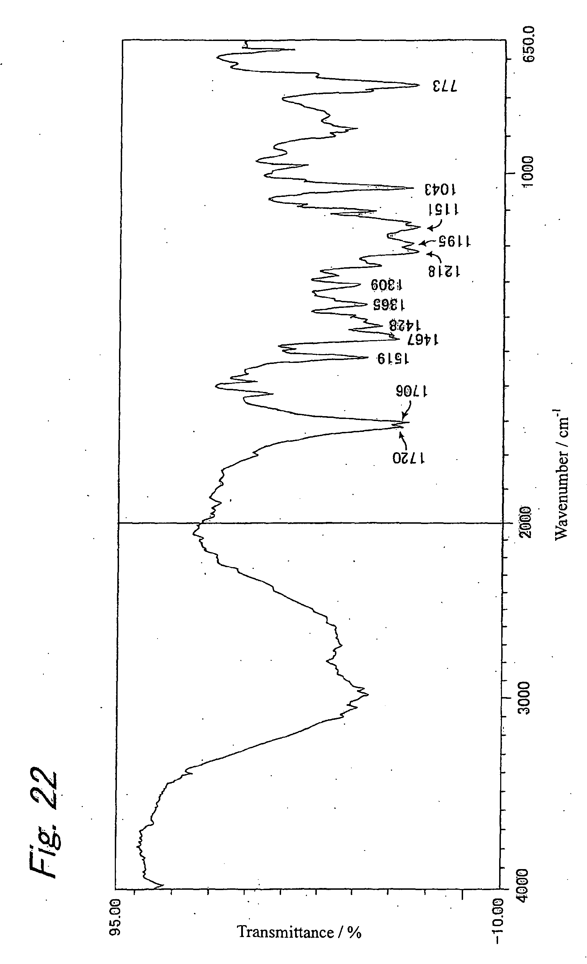

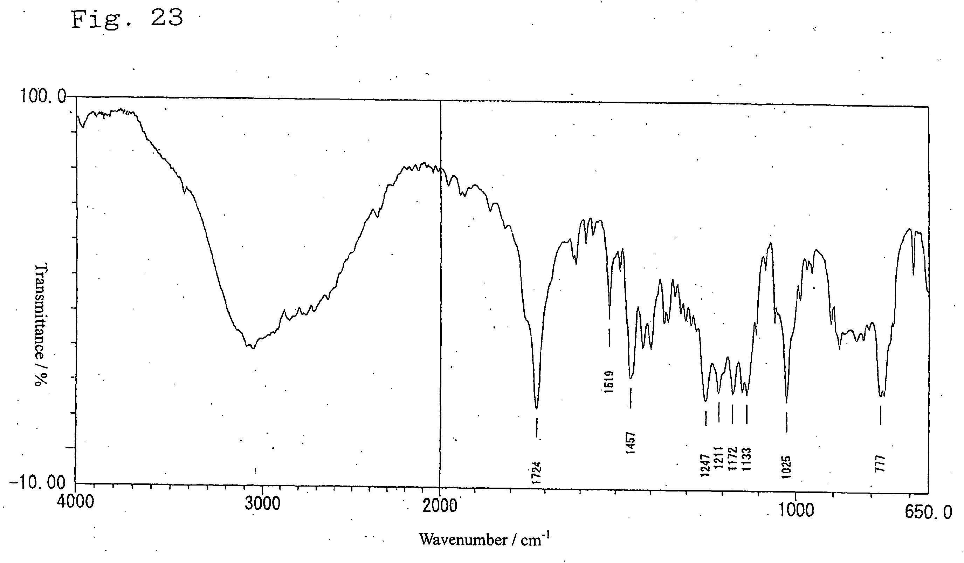

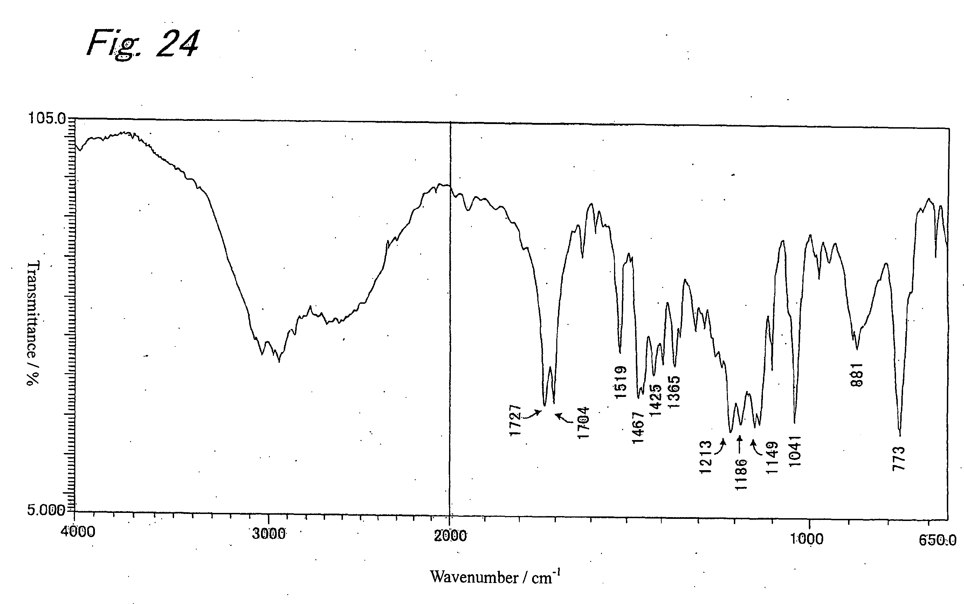

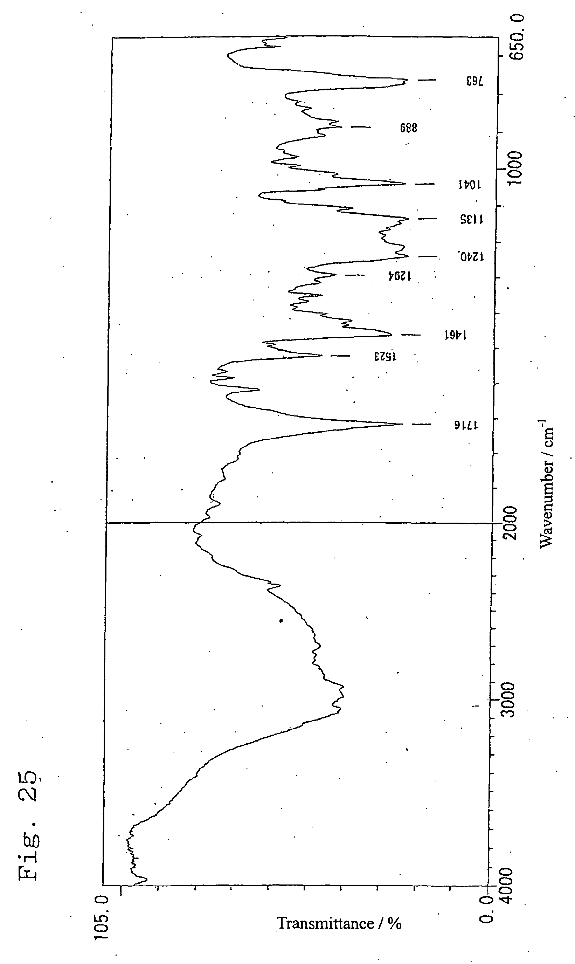

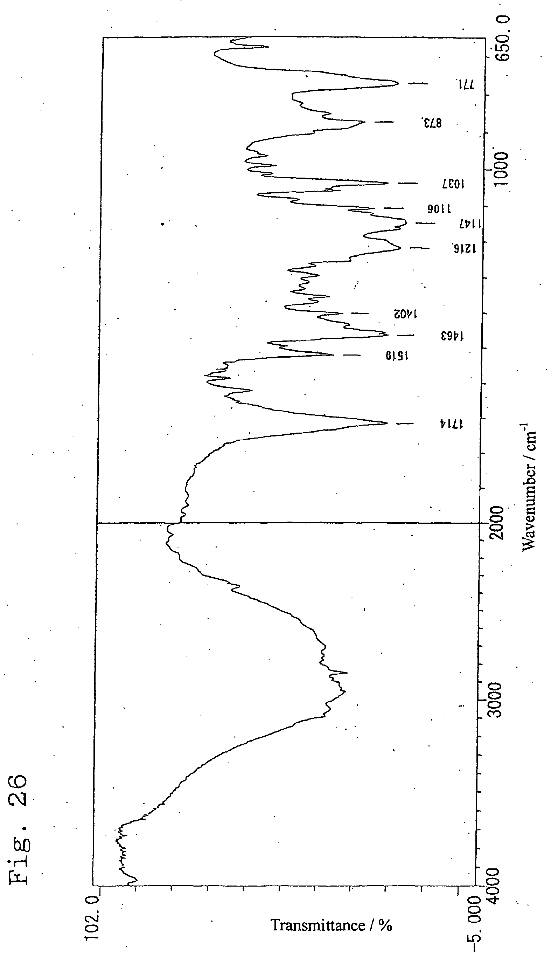

In other words, the present invention is as follows:
(1) An acid salt of 4-(1-((4-methylbenzothiophen-3-yl)methyl)benzimidazol-2-ylthio)butanoic acid.
(2) A crystal of an acid salt of 4-(1-((4-methylbenzothiophen-3-yl)methyl)benzimidazol-2-ylthio)butanoic acid.
(3) The salt according to (1), wherein the acid salt of 4-(1-((4-methylbenzothiophen-3-yl)methyl)benzimidazol-2-ylthio)butanoic acid is sulfate, hydrochloride, or methanesulfonate.
(4) The crystal according to (2), wherein the acid salt of 4-(1-((4-methylbenzothiophen-3-yl)methyl)benzimidazol-2-ylthio)butanoic acid is sulfate, hydrochloride, or methanesulfonate.
(5) A crystal of 4-(1-((4-methylbenzothiophen-3-yl)methyl)benzimidazol-2-ylthio)butanoic acid sulfate represented by formula (I) below: 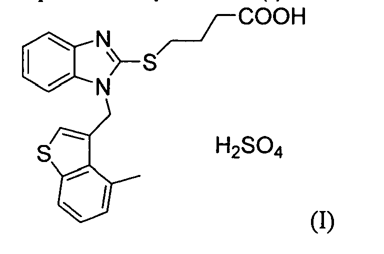
(6) The crystal of the sulfate according to (5) showing a powder X-ray diffraction pattern with characteristic peaks at diffraction angles 2θ of approximately 5.3°, 11.7°, 15.9°, 21.9°, 23.1°, and 27.5° (Crystal-SA).
(7) The crystal of the sulfate according to (5) showing a powder X-ray diffraction pattern approximately the same as that shown in Figure 1 (Crystal-SA).
(8) The crystal of the sulfate according to (5) showing a powder X-ray diffraction pattern with characteristic peaks at diffraction angles 2θ of approximately 11.4°, 13.8°, 15.9°, 16.6°, and 22.8° (Crystal-SB).
(9) The crystal of the sulfate according to (5) showing a powder X-ray diffraction pattern approximately the same as that shown in Figure 2 (Crystal-SB).
(10) The crystal of the sulfate according to (5) showing a powder X-ray diffraction pattern with characteristic peaks at diffraction angles 2θ of approximately 8.1°, 13.5°, 22.0°, 22.8°, and 24.2° (Crystal-SC).
(11) The crystal of the sulfate according to (5) showing a powder X-ray diffraction pattern approximately the same as that shown in Figure 3 (Crystal-SC).
(12) The crystal of the sulfate according to (5) showing a powder X-ray diffraction pattern with characteristic peaks at diffraction angles 2θ of approximately 12.9°, 21.2°, 22.9°, 24.7° and 27.3° (Crystal-SF).
(13) The crystal of the sulfate according to (5) showing a powder X-ray diffraction pattern approximately the same as that shown in Figure 4 (Crystal-SF).
(14) The crystal of the sulfate according to (5) showing a powder X-ray diffraction pattern with characteristic peaks at diffraction angles 2θ of approximately 12.2°, 13.5°, 18.5°, 22.4°, and 23.7° (Crystal-SG).
(15) The crystal of the sulfate according to (5) showing a powder X-ray diffraction pattern approximately the same as that shown in Figure 5 (Crystal-SG).
(16) The crystal of the sulfate according to (5) whose infrared absorption spectrum in potassium bromide exhibits peaks at wavenumbers of approximately 1715, 1456, 1203, 1067, 880, and 756 cm⁻¹ (Crystal-SA).
(17) The crystal of the sulfate according to (5) whose infrared absorption spectrum in potassium bromide exhibits the absorption pattern shown in Figure 6 (Crystal-SA).
(18) The crystal of the sulfate according to (5) whose infrared absorption spectrum in potassium bromide exhibits peaks at wavenumbers of approximately 1709, 1468, 1230, 1163, 1063, 862, and 754 cm⁻¹ (Crystal-SB).
(19) The crystal of the sulfate according to (5) whose infrared absorption spectrum in potassium bromide exhibits the absorption pattern shown in Figure 7 (Crystal-SB).
(20) The crystal of the sulfate according to (5) whose infrared absorption spectrum in potassium bromide exhibits peaks at wavenumbers of approximately 1705, 1459, 1325, 1238, 1152, 1065, 874, and 762 cm⁻¹ (Crystal-SC).
(21) The crystal of the sulfate according to (5) whose infrared absorption spectrum in potassium bromide exhibits the absorption pattern shown in Figure 8 (Crystal-SC).
(22) The crystal of the sulfate according to (5) whose infrared absorption spectrum in potassium bromide exhibits peaks at wavenumbers of approximately 1726, 1459, 1316, 1248, 1154, 1046, 869, and 768 cm⁻¹ (Crystal-SF).
(23) The crystal of the sulfate according to (5) whose infrared absorption spectrum in potassium bromide exhibits the absorption pattern shown in Figure 9 (Crystal-SF).
(24) The crystal of the sulfate according to (5) whose infrared absorption spectrum in potassium bromide exhibits peaks at wavenumbers of approximately 1709, 1467, 1317, 1234, 1152, 1065, 872, and 761 cm⁻¹ (Crystal-SG).
(25) The crystal of the sulfate according to (5) whose infrared absorption spectrum in potassium bromide exhibits the absorption pattern shown in Figure 10 (Crystal-SG).
(26) A crystal of 4-(1-((4-methylbenzothiophen-3-yl)methyl)benzimidazol-2-ylthio)butanoic acid hydrochloride represented by formula (II) below: 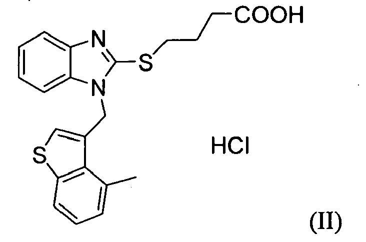
(27) The crystal of the hydrochloride according to (26) showing a powder X-ray diffraction pattern with characteristic peaks at diffraction angles 2θ of approximately 7.0°, 7.8°, 22.7°, 24.1 °, and 26.2° (Crystal-HA).
(28) The crystal of the hydrochloride according to (26) showing a powder X-ray diffraction pattern approximately the same as that shown in Figure 11 (Crystal-HA).
(29) The crystal of the hydrochloride according to (26) showing a powder X-ray diffraction pattern with characteristic peaks at diffraction angles 2θ of approximately 8.5°, 11.9°, 19.7°, and 21.2° (Crystal-HB).
(30) The crystal of the hydrochloride according to (26) showing a powder X-ray diffraction pattern approximately the same as that shown in Figure 12 (Crystal-HB).
(31) The crystal of the hydrochloride according to (26) showing a powder X-ray diffraction pattern with characteristic peaks at diffraction angles 2θ of approximately 7.9°, 11.5°, 14.4°, and 16.8° (Crystal-HC).
(32) The crystal of the hydrochloride according to (26) showing a powder X-ray diffraction pattern approximately the same as that shown in Figure 13 (Crystal-HC).
(33) The crystal of the hydrochloride according to (26) whose infrared absorption spectrum in potassium bromide exhibits peaks at wavenumbers of approximately 1712, 1465, 1253, 1184, 1116, 872, and 752 cm⁻¹ (Crystal-HA).
(34) The crystal of the hydrochloride according to (26) whose infrared absorption spectrum in potassium bromide exhibits the absorption pattern shown in Figure 14 (Crystal-HA).
(35) The crystal of the hydrochloride according to (26) whose infrared absorption spectrum in potassium bromide exhibits peaks at wavenumbers of approximately 1708, 1458, 1242, 1103, 1028, 881, and 760 cm⁻¹ (Crystal-HB).
(36) The crystal of the hydrochloride according to (26) whose infrared absorption spectrum in potassium bromide exhibits the absorption pattern shown in Figure 15 (Crystal-HB).
(37) The crystal of the hydrochloride according to (26) whose infrared absorption spectrum in potassium bromide exhibits peaks at wavenumbers of approximately 1707, 1461, 1182, 1032, and 754 cm⁻¹ (Crystal-HC).
(38) The crystal of the hydrochloride according to (26) whose infrared absorption spectrum in potassium bromide exhibits the absorption pattern shown in Figure 16 (Crystal-HC).
(39) A crystal of 4-(1-((4-methylbenzothiophen-3-yl)methyl)benzimidazol-2-ylthio)butanoic acid methanesulfonate represented by formula (III) below: 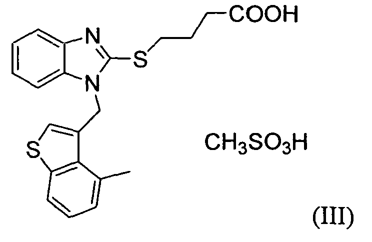
(40) The crystal of the methanesulfonate according to (39) showing a powder X-ray diffraction pattern with characteristic peaks at diffraction angles 2θ of approximately 6.6°, 18.4°, and 21.3° (Crystal-MA).
(41) The crystal of the methanesulfonate according to (39) showing a powder X-ray diffraction pattern approximately the same as that shown in Figure 17 (Crystal-MA).
(42) The crystal of the methanesulfonate according to (39) showing a powder X-ray diffraction pattern with characteristic peaks at diffraction angles 2θ of approximately 8.3°, 9.6°, 13.7°, and 25.0° (Crystal-MB).
(43) The crystal of the methanesulfonate according to (39) showing a powder X-ray diffraction pattern approximately the same as that shown in Figure 18 (Crystal-MB).
(44) The crystal of the methanesulfonate according to (39) showing a powder X-ray diffraction pattern with characteristic peaks at diffraction angles 2θ of approximately 6.1 °, 12.4°, 18.2°, 20.7°, and 24.2° (Crystal-MC).
(45) The crystal of the methanesulfonate according to (39) showing a powder X-ray diffraction pattern approximately the same as that shown in Figure 19 (Crystal-MC).
(46) The crystal of the methanesulfonate according to (39) showing a powder X-ray diffraction pattern with characteristic peaks at diffraction angles 2θ of approximately 9.1°, 12.6°, 14.7°, and 25.3° (Crystal-MD).
(47) The crystal of the methanesulfonate according to (39) showing a powder X-ray diffraction pattern approximately the same as that shown in Figure 20 (Crystal-MD).
(48) The crystal of the methanesulfonate according to (39) showing a powder X-ray diffraction pattern with characteristic peaks at diffraction angles 2θ of approximately 8.7°, 18.9°, and 22.6° (Crystal-ME).
(49) The crystal of the methanesulfonate according to (39) showing a powder X-ray diffraction pattern approximately the same as that shown in Figure 21 (Crystal-ME).
(50) The crystal of the methanesulfonate according to (39) whose infrared absorption spectrum in potassium bromide exhibits peaks at wavenumbers of approximately 1720, 1707, 1467, 1309,1218, 1196, 1151, 1043, and 773 cm⁻¹ (Crystal-MA).
(51) The crystal of the methanesulfonate according to (39) whose infrared absorption spectrum in potassium bromide exhibits the absorption pattern shown in Figure 22 (Crystal-MA).
(52) The crystal of the methanesulfonate according to (39) whose infrared absorption spectrum in potassium bromide exhibits peaks at wavenumbers of approximately 1724, 1457,1247, 1211, 1173, 1025, and 777 cm⁻¹ (Crystal-MB).
(53) The crystal of the methanesulfonate according to (39) whose infrared absorption spectrum in potassium bromide exhibits the absorption pattern shown in Figure 23 (Crystal-MB).
(54) The crystal of the methanesulfonate according to (39) whose infrared absorption spectrum in potassium bromide exhibits peaks at wavenumbers of approximately 1728, 1705, 1468, 1365, 1213, 1186, 1149, 1041, 881, and 773 cm⁻¹ (Crystal-MC).
(55) The crystal of the methanesulfonate according to (39) whose infrared absorption spectrum in potassium bromide exhibits the absorption pattern shown in Figure 24 (Crystal-MC).
(56) The crystal of the methanesulfonate according to (39) whose infrared absorption spectrum in potassium bromide exhibits peaks at wavenumbers of approximately 1716, 1461, 1240, 1136, 1041, 1041, and 764 cm⁻¹ (Crystal-MD).
(57) The crystal of the methanesulfonate according to (39) whose infrared absorption spectrum in potassium bromide exhibits the absorption pattern shown in Figure 25 (Crystal-MD).
(58) The crystal of the methanesulfonate according to (39) whose infrared absorption spectrum in potassium bromide exhibits peaks at wavenumbers of approximately 1714, 1464, 1216, 1037, 874, and 771 cm⁻¹ (Crystal-ME).
(59) The crystal of the methanesulfonate according to (39) whose infrared absorption spectrum in potassium bromide exhibits the absorption pattern shown in Figure 26 (Crystal-ME).
(60) A pharmaceutical composition comprising the acid salt of 4-(1-((4-methylbenzothiophen-3-yl)methyl)benzimidazol-2-ylthio)butanoic acid or the crystal thereof described in (1) or (2) as an active ingredient.
(61) The pharmaceutical composition according to (60), wherein the acid salt is sulfate, hydrochloride, or methanesulfonate.
(62) A chymase inhibitory agent comprising the acid salt of 4-(1-((4-methylbenzothiophen-3-yl)methyl)benzimidazol-2-ylthio)butanoic acid or the crystal thereof described in (1) or (2) as an active ingredient.
(63) The chymase inhibitory agent according to (62) wherein the acid salt is sulfate, hydrochloride, or methanesulfonate.
(64) A preventive and/or therapeutic agent for inflammatory diseases, allergic diseases, respiratory diseases, circulatory diseases, or bone/cartilage metabolic diseases comprising the acid salt of 4-(1-((4-methylbenzothiophen-3-yl)methyl)benzimidazol-2-ylthio)butanoic acid or the crystal thereof described in (1) or (2) as an active ingredient.
(65) The preventive and/or therapeutic agent according to (64), wherein the acid salt is sulfate, hydrochloride, or methanesulfonate.
(66) A pharmaceutical composition comprising a crystal or a mixture of two or more forms of crystals selected from the crystals of the sulfate described in (5) to (25) as an active ingredient.
(67) A chymase inhibitory agent comprising a crystal or a mixture of two or more forms of crystals selected from the crystals of the sulfate described in (5) to (25) as an active ingredient.
(68) A preventive and/or therapeutic agent for inflammatory diseases, allergic diseases, respiratory diseases, circulatory diseases, or bone/cartilage metabolic diseases comprising a crystal or a mixture of two or more forms of crystals selected from the crystals of the sulfate described in (5) to (25) as an active ingredient.
(69) A pharmaceutical composition comprising a crystal or a mixture of two or more forms of crystals selected from the crystals of the hydrochloride described in (26) to (38) as an active ingredient.
(70) A chymase inhibitory agent comprising a crystal or a mixture of two or more forms of crystals selected from the crystals of the hydrochloride described in (26) to (38) as an active ingredient.
(71) A preventive and/or therapeutic agent for inflammatory diseases, allergic diseases, respiratory diseases, circulatory diseases, or bone/cartilage metabolic diseases comprising a crystal or a mixture of two or more forms of crystals selected from the crystals of the hydrochloride described in (26) to (38) as an active ingredient.
(72) A pharmaceutical composition comprising a crystal or a mixture of two or more forms of crystals selected from the crystals of the methanesulfonate described in (39) to (59) as an active ingredient.
(73) A chymase inhibitory agent comprising a crystal or a mixture of two or more forms of crystals selected from the crystals of the methanesulfonate described in (39) to (59) as an active ingredient.
(74) A preventive and/or therapeutic agent for inflammatory diseases, allergic diseases, respiratory diseases, circulatory diseases, or bone/cartilage metabolic diseases comprising a crystal or a mixture of two or more forms of crystals selected from the crystals of the methanesulfonate described in (39) to (59) as an active ingredient.
(75) A pharmaceutical composition comprising a crystal or a mixture of two or more forms of crystals selected from the crystals described in (5) to (59) as an active ingredient.
(76) A chymase inhibitory agent comprising a crystal or a mixture of two or more forms of crystals selected from the crystals described in (5) to (59) as an active ingredient.
(77) A preventive and/or therapeutic agent for inflammatory diseases, allergic diseases, respiratory diseases, circulatory diseases, or bone/cartilage metabolic diseases comprising a crystal or a mixture of two or more forms of crystals selected from the crystals described in (5) to (59) as an active ingredient.

### Brief Description of the Drawings

Figure 1 shows the XRD pattern of crystal-SA of the sulfate according to the present invention; Figure 2 shows the XRD pattern of crystal-SB of the sulfate according to the present invention; Figure 3 shows the XRD pattern of crystal-SC of the sulfate according to the present invention; Figure 4 shows the XRD pattern of crystal-SF of the sulfate according to the present invention; Figure 5 shows the XRD pattern of crystal-SG of the sulfate according to the present invention; Figure 6 shows the IR spectrum of crystal-SA of the sulfate according to the present invention; Figure 7 shows the IR spectrum of the crystal-SB of the sulfate according to the present invention;
Figure 8 shows the IR spectrum of the crystal-SC of the sulfate according to the present invention; Figure 9 shows the IR spectrum of crystal-SF of the sulfate according to the present invention; Figure 10 shows the IR spectrum of crystal-SG of the sulfate according to the present invention; Figure 11 shows the XRD pattern of crystal-HA of the hydrochloride according to the present invention; Figure 12 shows the XRD pattern of crystal-HB of the hydrochloride according to the present invention; Figure 13 shows the XRD pattern of crystal-HC of the hydrochloride according to the present invention;
Figure 14 shows the IR spectrum of crystal-HA of the hydrochloride according to the present invention; Figure 15 shows the IR spectrum of crystal-HB of the hydrochloride according to the present invention; Figure 16 shows the IR spectrum of crystal-HC of the hydrochloride according to the present invention; Figure 17 shows the XRD pattern of crystal-MA of the methanesulfonate according to the present invention; Figure 18 shows the XRD pattern of crystal-MB of the methanesulfonate according to the present invention; Figure 19 shows the XRD pattern of crystal-MC of the methanesulfonate according to the present invention; Figure 20 shows the XRD pattern of crystal-MD of the methanesulfonate according to the present invention; Figure 21 shows the XRD pattern of crystal-ME of the methanesulfonate according to the present invention;
Figure 22 shows the IR spectrum of crystal-MA of the methanesulfonate according to the present invention; Figure 23 shows the IR spectrum of crystal-MB of the methanesulfonate according to the present invention; Figure 24 shows the IR spectrum of crystal-MC of the methanesulfonate according to the present invention; Figure 25 shows the IR spectrum of crystal-MD of the methanesulfonate according to the present invention; and Figure 26 shows the IR spectrum of crystal-ME of the methanesulfonate according to the present invention.

### Best Mode for Carrying Out the Invention

Sulfate, hydrochloride, or methanesulfonate of 4-(1-((4-methylbenzothiophen-3-yl)methyl)benzimidazol-2-ylthio)butanoic acid according to the present invention is a salt with a counter anion, that is an acid salt, of 4-(1-((4-methylbenzothiophen-3-yl)methyl)benzimidazol-2-ylthio)butanoic acid. The acid salts of 4-(1-((4-methylbenzothiophen-3-yl)methyl)benzimidazol-2-ylthio)butanoic acid according to the present invention other than sulfate, hydrochloride, or methanesulfonate include phosphate and maleate.

The acid salt, such as sulfate, hydrochloride, and methanesulfonate, of 4-(1-((4-methylbenzothiophen-3-yl)methyl)benzimidazol-2-ylthio)butanoic acid and the crystal thereof according to the present invention strongly inhibit human chymase activity. Specifically, the IC₅₀ is 1 nM or higher and 10 nM or lower. The acid salt, such as sulfate, hydrochloride, and methanesulfonate, of 4-(1-((4-methylbenzothiophen-3-yl)methyl)benzimidazol-2-ylthio)butanoic acid and the crystal thereof, which have such an excellent inhibitory activity on human chymase and high solubility in aqueous solvents, can be used as preventive and/or therapeutic agents clinically applicable to patients with various diseases.

The crystals according to the present invention are characterized by powder X-ray diffraction patterns and/or infrared absorption peaks in potassium bromide or the like. These crystals exhibit characteristic powder X-ray diffraction patterns (XRD) and each crystal has peaks at specific 2θ values. Each of these crystals exhibit a characteristic absorption pattern also in infrared spectrum (IR).

Crystal-SA of the sulfate according to the present invention shows a powder X-ray diffraction pattern with diffraction angles 2θ of approximately 5.3°, 11.7°, 15.9°, 21.9°, 23.1°, and 27.5°. More specifically, the powder X-ray diffraction pattern of crystal-SA has characteristic peaks listed in Table 1 (See Figure 1). For the intensity in the powder X-ray diffraction pattern listed in Tables, Iₘₐₓ represents the intensity of the most intense peak for each crystal and I represents the intensity of each peak. Each 2θ value in a powder X-ray diffraction pattern may vary by approximately 0.5° depending on the sample state and measurement conditions. For powder X-ray diffractometry, due to the nature of this technique, an overall pattern is important to identify crystals, and each relative intensity may somewhat vary depending on the direction of crystal growth, particle size, and measurement conditions and thus should not be considered as a strict value.

**Table 1 (Crystal-SA)**

| Diffraction angle (2θ/°) | Intensity (I/Iₘₐₓ× 100) |
|---|---|
| 5.3 | 35 |
| 11.7 | 88 |
| 15.9 | 62 |
| 21.9 | 91 |
| 23.1 | 100 |
| 27.5 | 42 |

Crystal-SB of the sulfate according to the present invention shows a powder X-ray diffraction pattern with diffraction angles 2θ of approximately 11.4°, 13.8°, 15.9°, 16.6°, and 22.8°. More specifically, the powder X-ray diffraction pattern of crystal-SB has characteristic peaks listed in Table 2 (See Figure 2).

**Table 2 (Crystal-SB)**

| Diffraction angle (2θ/°) | Intensity (I/Iₘₐₓ× 100) |
|---|---|
| 11.4 | 26 |
| 13.8 | 34 |
| 15.9 | 40 |
| 16.6 | 59 |
| 22.8 | 100 |

Crystal-SC of the sulfate according to the present invention shows a powder X-ray diffraction pattern with diffraction angles 2θ of approximately 8.1°, 13.5°, 22.0°, 22.8°, and 24.2°. More specifically, the powder X-ray diffraction pattern of crystal-SC has characteristic peaks listed in Table 3 (See Figure 3).

**Table 3 (Crystal-SC)**

| Diffraction angle (2θ/°) | Intensity (I/Iₘₐₓ× 100) |
|---|---|
| 8.1 | 34 |
| 13.5 | 32 |
| 22.0 | 39 |
| 22.8 | 100 |
| 24.2 | 78 |

Crystal-SF of the sulfate according to the present invention shows a powder X-ray diffraction pattern with diffraction angles 2θ of approximately 12.9°, 21.2°, 22.9°, 24.7°, and 27.3°. More specifically, the powder X-ray diffraction pattern of crystal-SF has characteristic peaks listed in Table 4 (See Figure 4).

**Table 4 (Crystal-SF)**

| Diffraction angle (2θ/°) | Intensity (I/Iₘₐₓ× 100) |
|---|---|
| 12.9 | 100 |
| 21.2 | 82 |
| 22.9 | 61 |
| 24.7 | 90 |
| 27.3 | 34 |

Crystal-SG of the sulfate according to the present invention shows a powder X-ray diffraction pattern with diffraction angles 2θ of approximately 12.2°, 13.5°, 18.5°, 22.4°, and 23.7°. More specifically, the powder X-ray diffraction pattern of crystal-SG has characteristic peaks listed in Table 5 (See Figure 5).

**Table 5 (Crystal-SG)**

| Diffraction angle (2θ/°) | Intensity (I/Iₘₐₓ× 100) |
|---|---|
| 12.2 | 36 |
| 13.5 | 37 |
| 18.5 | 66 |
| 22.4 | 100 |
| 23.7 | 92 |

The infrared spectrum of crystal-SA of the sulfate according to the present invention shows peaks at wavenumbers of approximately 1715, 1456, 1203, 1067, 880, and 756 cm⁻¹ (See Figure 6).

The infrared spectrum of crystal-SB of the sulfate according to the present invention shows peaks at wavenumbers of approximately 1709, 1468, 1230, 1163, 1063, 862, and 754 cm⁻¹ (See Figure 7).

The infrared spectrum of crystal-SC of the sulfate according to the present invention shows peaks at wavenumbers of approximately 1705, 1459, 1325, 1238, 1152, 1065, 874, and 762 cm⁻¹ (See Figure 8).

The infrared spectrum of crystal-SF of the sulfate according to the present invention shows peaks at wavenumbers of approximately 1726, 1459, 1316, 1248, 1154, 1046, 869, and 768 cm⁻¹ (See Figure 9).

The infrared spectrum of crystal-SG of the sulfate according to the present invention shows peaks at wavenumbers of approximately 1709, 1467, 1317, 1234, 1152, 1065, 872, and 761 cm⁻¹ (See Figure 10).

The wavenumbers measured in infrared spectroscopy in the present invention may vary by approximately 5 cm⁻¹ depending on measurement conditions, sample conditions, or the like.

Crystal-HA of the hydrochloride according to the present invention shows a powder X-ray diffraction pattern with diffraction angles 2θ of approximately 7.0°, 7.8°, 22.7°, 24.1°, and 26.2°. More specifically, the powder X-ray diffraction pattern of crystal-HA has characteristic peaks listed in Table 6 (See Figure 11).

**Table 6 (Crystal-HA)**

| Diffraction angle (2θ/°) | Intensity (I/Iₘₐₓ× 100) |
|---|---|
| 7.0 | 68 |
| 7.8 | 60 |
| 22.7 | 100 |
| 24.1 | 90 |
| 26.2 | 78 |

Crystal-HB of the hydrochloride according to the present invention shows a powder X-ray diffraction pattern with diffraction angles 2θ of approximately 8.5, 11.9°, 19.7°, and 21.2°. More specifically, the powder X-ray diffraction pattern of crystal-HB has characteristic peaks listed in Table 7 (See Figure 12).

**Table 7 (Crystal-HB)**

| Diffraction angle (2θ/°) | Intensity (I/Iₘₐₓ× 100) |
|---|---|
| 8.5 | 100 |
| 11.9 | 49 |
| 19.7 | 100 |
| 21.2 | 69 |

Crystal-HC of the hydrochloride according to the present invention shows a powder X-ray diffraction pattern with diffraction angles 2θ of approximately 7.9°, 11.5°, 14.4°, and 16.8°. More specifically, the powder X-ray diffraction pattern of crystal-HC has characteristic peaks listed in Table 8 (See Figure 13).

**Table 8 (Crystal-HC)**

| Diffraction angle (2θ/°) | Intensity (I/Iₘₐₓ× 100) |
|---|---|
| 7.9 | 49 |
| 11.5 | 52 |
| 14.4 | 56 |
| 16.8 | 100 |

The infrared spectrum of crystal-HA of the hydrochloride according to the present invention shows peaks at wavenumbers of approximately 1712, 1465, 1253, 1184, 1116, 872, and 752 cm⁻¹ (See Figure 14).

The infrared spectrum of crystal-HB of the hydrochloride according to the present invention shows peaks at wavenumbers of approximately 1708, 1458, 1242, 1103, 1028, 881, and 760 cm⁻¹ (See Figure 15).

The infrared spectrum of crystal-HC of the hydrochloride according to the present invention shows peaks at wavenumbers of approximately 1707, 1461, 1182, 1032, and 754 cm- (See Figure 16).

Crystal-MA of the methanesulfonate according to the present invention shows a powder X-ray diffraction pattern with diffraction angles 2θ of approximately 6.6°, 18.4°, and 21.3°. More specifically, the powder X-ray diffraction pattern of crystal-MA has characteristic peaks listed in Table 9 (See Figure 17).

**Table 9 (Crystal-MA)**

| Diffraction angle (2θ/°) | Intensity (1/Iₘₐₓ× 100) |
|---|---|
| 6.6 | 100 |
| 18.4 | 19 |
| 21.3 | 18 |

Crystal-MB of the methanesulfonate according to the present invention shows a powder X-ray diffraction pattern with diffraction angles 2θ of approximately 8.3°, 9.6°, 13.7°, and 25.0°. More specifically, the powder X-ray diffraction pattern of crystal-MB has characteristic peaks listed in Table 10 (See Figure 18).

**Table 10 (Crystal-MB)**

| Diffraction angle (2θ/°) | Intensity (I/Iₘₐₓ×100) |
|---|---|
| 8.3 | 28 |
| 9.6 | 49 |
| 13.7 | 80 |
| 25.0 | 100 |

Crystal-MC of the methanesulfonate according to the present invention shows a powder X-ray diffraction pattern with diffraction angles 2θ of approximately 6.1°, 12.4°, 18.2°, 20.7°, and 24.2°. More specifically, the powder X-ray diffraction pattern of crystal-MC has characteristic peaks listed in Table 11 (See Figure 19).

**Table 11 (Crystal-MC)**

| Diffraction angle (2θ/°) | Intensity (I/Iₘₐₓ× 100) |
|---|---|
| 6.1 | 100 |
| 12.4 | 26 |
| 18.2 | 28 |
| 20.7 | 33 |
| 24.2 | 31 |

Crystal-MD of the methanesulfonate according to the present invention shows a powder X -ray diffraction pattern with diffraction angles 2θ of approximately 9.1°, 12.6°, 14.7°, and 25.3°. More specifically, the powder X-ray diffraction pattern of crystal-MD has characteristic peaks shown in Table 12 (See Figure 20).

**Table 12 (Crystal-MD)**

| Diffraction angle (2θ/°) | Intensity (I/Iₘₐₓ× 100) |
|---|---|
| 9.1 | 41 |
| 12.6 | 70 |
| 14.7 | 70 |
| 25.3 | 100 |

Crystal-ME of the methanesulfonate according to the present invention shows a powder X-ray diffraction pattern with diffraction angles 2θ of approximately 8.7°, 18.9°, and 22.6°. More specifically, the powder X-ray diffraction pattern of crystal-ME has characteristic peaks listed in Table 13 (See Figure 21).

**Table 13 (Crystal-ME)**

| Diffraction angle (2θ/°) | Intensity (I/Iₘₐₓ× 100) |
|---|---|
| 8.7 | 100 |
| 18.9 | 54 |
| 22.6 | 67 |

The infrared spectrum of crystal-MA of the methanesulfonate according to the present invention shows peaks at wavenumbers of approximately 1720, 1707, 1467, 1309, 1218, 1196, 1151, 1043, and 773 cm⁻¹ (See Figure 22).

The infrared spectrum of crystal-MB of the methanesulfonate according to the present invention shows peaks at wavenumbers of approximately 1724, 1457, 1247, 1211, 1173, 1025, and 777 cm⁻¹ (See Figure 23).

The infrared spectrum of crystal-MC of the methanesulfonate according to the present invention shows peaks at wavenumbers of approximately 1728, 1705, 1468, 1365, 1213, 1186, 1149, 1041, 881, and 773 cm⁻¹ (See Figure 24).

The infrared spectrum of crystal-MD of the methanesulfonate according to the present invention shows peaks at wavenumbers of approximately 1716, 1461, 1240, 1136, 1041, 1041, and 764 cm⁻¹ (See Figure 25).

The infrared spectrum of crystal-ME of the methanesulfonate according to the present invention shows peaks at wavenumbers of approximately 1714, 1464, 1216, 1037, 874, and 771 cm⁻¹ (See Figure 25).

The three acid salts and the crystals thereof according to the present invention can be obtained by various manufacturing methods, respectively, but typical examples will be described below.

The precursor of acid salts, 4-(1-((4-methylbenzothiophen-3-yl)methyl)benzimidazol-2-ylthio)butanoic acid, can be synthesized by the method described in Patent Document 3, or Document 4, or the like. For example, methyl 4-(1-((4-methylbenzothiophen-3-yl)methyl)benzimidazol-2-ylthio)butanoate can be obtained by a coupling reaction of 3-bromomethyl-4-methylbenzothiophene with methyl 4-(benzimidazol-2-ylthio)butanoate in the presence of a base such as a tertiary amine in a hydrocarbon solvent such as toluene. 4-(1-((4-methylbenzothiophen-3-yl)methyl)benzimidazol-2-ylthio)butanoic acid can be obtained by, following hydrolysis with an aqueous solution of sodium hydroxide or the like in tetrahydrofuran as solvent and subsequent neutralization.

The acid salt of 4-(1-((4-methylbenzothiophen-3-yl)methyl)benzimidazol-2-ylthio)butanoic acid according to the present invention can be obtained by adding the acid to a solution consisting of 4-(1-((4-methylbenzothiophen-3-yl)methyl)benzimidazol-2-ylthio)butanoic acid and a solvent. (The acid may be added as a solution in a solvent. For hydrochloride, gaseous hydrogen chloride may be blown in.) The state of product solution depends on conditions such as solvent, concentration, and temperature: the acid salt is obtained in a solution state when the solution is unsaturated; while the acid salt may be sometimes immediately precipitated as crystals or maintained in a solution state when the solution is supersaturated. Those skilled in the art could obtain any of supersaturated solutions and unsaturated solutions by adjusting conditions such as solvent, concentration, and temperature as appropriate. When the acid salt is in a solution state, the salt can be precipitated as crystals by concentration, cooling, addition of a poor solvent, or another method.

The order in stability among the crystal forms of each acid salt can be examined by solvent-mediated transformation of a mixture of crystals, but the order may change depending on the solvent and temperature. For the sulfate, the stability of crystal-SC, crystal-SA, and crystal-SB declines in this order in a solvent mainly composed of a solvent in which these crystals are highly soluble (for example, acetic acid). The stability of crystal-SF is close to that of crystal-SA, and the order of their stability is reversed depending on temperature. Crystal-SG is less stable than crystal-SF, and it is confirmed that crystal-SG can be transformed into crystal-SF or directly transformed to crystal-SC. Crystal-SC is more stable than the other four crystal forms. For the hydrochloride, crystal-HB is more stable than the other crystal forms. For the methanesulfonate, the stability depends on solvent, and no general conclusion can be drawn on which crystal form is the most stable.

All of the sulfate and crystal-SA, crystal-SB, crystal-SC, crystal-SF, and crystal-SG thereof can be manufactured by the above methods. The solvents used include acetone, anisole, ethanol, formic acid, ethyl formate, cumene, acetic acid, isobutyl acetate, isopropyl acetate, ethyl acetate, butyl acetate, propyl acetate, methyl acetate, propanoic acid, diethyl ether, t-butyl methyl ether, 1-butanol, 2-butanol, 1-propanol, 2-propanol, heptane, 1-pentanol, 4-methyl-2-pentanone, 2-butanone, 3-methyl-1-butanol, 2-methyl-1-propanol, tetrahydrofuran, acetonitrile, cyclohexane, 1,2-dimethoxyethane, 1,4-dioxane, 2-ethoxyethanol, hexane, pentane, methanol, 2-ethoxymethanol, methylcyclohexane, tetralin, toluene, xylene, water, and a mixture of two or more selected from these. Solvents preferable from economical and industrial viewpoints include acetic acid, methyl acetate, ethyl acetate, propyl acetate, isopropyl acetate, butyl acetate, 4-methyl-2-pentanone, 2-butanone, acetone, tetrahydrofuran, t-butyl methyl ether, 1,4-dioxane, acetonitrile, hexane, cyclohexane, heptane, toluene, xylene, methanol, ethanol, 1-propanol, 2-propanol, and a mixture of two or more selected from these. More preferably, the solvent is acetic acid, ethyl acetate, isopropyl acetate, butyl acetate, 4-methyl-2-pentanone, 2-butanone, acetone, tetrahydrofuran, acetonitrile, hexane, cyclohexane, heptane, toluene, xylene, or a mixture of two or more selected from these.

To obtain the sulfate and crystal-SA, crystal-SB, crystal-SC, crystal-SF, or crystal-SG thereof, the amount of solvent to be used is, although not particularly limited to, preferably 5 to 100 times, more preferably 50 times or less, further preferably 20 times or less. Here, an amount of 1 time means that 1 mL of a solvent is used for 1 g of the source material, (4-(1-((4-methylbenzothiophen-3-yl)methyl)benzimidazol-2-ylthio)butanoic acid or its sulfate. In order to reduce the amount of solvent, it is preferred to use a solvent in which 4-(1-((4-methylbenzothiophen-3-yl)methyl)benzimidazol-2-ylthio)butanoic acid or sulfate thereof is highly soluble, and such solvent includes acetic acid and tetrahydrofuran.

To obtain crystal-SA, crystal-SB, crystal-SC, or crystal-SF of the sulfate by crystallization from a solution, it is effective to add seed crystals in the same crystal form as that of the desired crystal. The amount of seed crystals is generally about 0.0 1 % by weight to about 20% by weight, preferably 0.1 % by weight to 10% by weight of the source material, 4-(1-((4-methylbenzothiophen-3-yl)methyl)benzimidazol-2-ylthio)butanoic acid or its sulfate, and seed crystals are preferably crushed in advance. Upon addition of seed crystals, the solution is required to be supersaturated for the desired species.

Crystal-SF of the sulfate is relatively easily obtained. For example, crystals in this form can be crystallized with good reproducibility by adding sulfuric acid to a tetrahydrofuran solution of 4-(1-((4-methylbenzothiophen-3-yl)methyl)benzimidazol-2-ylthio)butanoic acid, or by cooling an acetic acid solution of the sulfate. In this procedure, adding seed crystals of crystal-SF is more preferred.

Crystal-SG of the sulfate can be obtained, for example, by adding, to an acetic acid solution of 4-(1-((4-methylbenzothiophen-3-yl)methyl)benzimidazol-2-ylthio)butanoic acid sulfate that has been seeded with crystals in the crystal-SC-form in advance, a nonpolar solvent such as heptane in a short time to solidify the sulfate. Alternatively, crystal-SG can be crystallized by dissolving the sulfate in a solvent such as acetic acid, a mixed solvent of acetic acid and 2-butanone, a mixed solvent of acetic acid and 4-methyl-2-pentanone or a like at about 60°C to about 80°C to prepare a supersaturated solution, followed by adding crystal-SC to the solution as seed crystals and standing the solution at almost the same temperature.

Crystal-SA or crystal-SB of the sulfate can be crystallized, for example, by cooling a solution of 4-(1-((4-methylbenzothiophen-3-yl)methyl)benzimidazol-2-ylthio)butanoic acid sulfate in a mixed solvent of acetic acid and 2-butanone, a mixed solvent of acetic acid and 4-methyl-2-pentanone or a like. Here, when the proportion of acetic acid in the solvent is less than 50%, the sulfate tends to crystallize as crystal-SA, whereas when the proportion is 50% or higher, it tends to crystallize as crystal-SB. In this method, addition of seed crystals in the desired crystal form is more preferred.

There is no particular restriction on the temperature, stirring condition, and time before filtration after the start of crystallization of crystal-SA, crystal-SB, crystal-SF, or crystal-SG of the sulfate. Since these conditions may affect the yield, chemical purity, particle size, or the like of crystals, however, it is preferable to adjust these conditions in combination according to the purpose.

Although crystal-SC of the sulfate is not always readily crystallized by the above methods, it can be obtained by suspending another form of crystals selected from crystal-SA, crystal-SB, crystal-SF, crystal-SG, or a mixture of two or more forms of crystals selected from these, in a solvent to perform solvent-mediated transformation.

In solvent-mediated transformation of the sulfate from a crystal form other than crystal-SC to crystal-SC, the solvent to be used includes acetone, anisole, ethanol, formic acid, ethyl formate, cumene, acetic acid, isobutyl acetate, isopropyl acetate, ethyl acetate, butyl acetate, propyl acetate, methyl acetate, propanoic acid, diethyl ether, t-butylmethyl ether, 1-butanol, 2-butanol, 1-propanol, 2-propanol, heptane, 1-pentanol, 4-methyl-2-pentanone, 2-butanone, 3-methyl-1-butanol, 2-methyl-1-propanol, tetrahydrofuran, acetonitrile, cyclohexane, 1,2-dimethoxyethane, 1,4-dioxane, 2-ethoxyethanol, hexane, pentane, methanol, 2-ethoxymethanol, methylcyclohexane, tetralin, toluene, xylene, water, and a mixture of two or more selected from these. The solvent preferable from economical and industrial viewpoints includes acetic acid, methyl acetate, ethyl acetate, propyl acetate, isopropyl acetate, butyl acetate, 4-methyl-2-pentanone, 2-butanone, acetone, tetrahydrofuran, t-butylmethyl ether, 1,4-dioxane, acetonitrile, hexane, cyclohexane, heptane, toluene, xylene, methanol, ethanol, 1-propanol, 2-propanol, and a mixture of two or more selected from these. More preferably, the solvent is acetic acid, ethyl acetate, isopropyl acetate, butyl acetate, 4-methyl-2-pentanone, 2-butanone, acetone, tetrahydrofuran, acetonitrile, hexane, cyclohexane, heptane, toluene, xylene, or a mixture of two or more selected from these.

In order to reduce the time required for solvent-mediated transformation of the sulfate from a crystal form other than crystal-SC to crystal-SC, addition of crystal-SC as seed crystals is effective. The amount of seed crystals is generally about 0.01% to about 20%, preferably 0.1 % to 10% of another form of crystals to be transformed, and the seed crystals are preferably crushed in advance. The seed crystals may be mixed with the source crystals in advance or added to the suspension afterwards.

The temperature in solvent-mediated transformation of the sulfate from a crystal form other than crystal-SC to crystal-SC is preferably 100°C or lower to avoid decomposition of 4-(1-((4-methylbenzothiophen-3-yl)methyl)benzimidazol-2-ylthio)butanoic acid sulfate, although the transformation rate increases with the temperature. The amount of solvent used for transformation must be determined so that the system is in a suspended state at the temperature for transformation. The amount is generally 2 times to 100 times, preferably 50 times or less, and more preferably 20 times or less of the amount of another form of crystals to be transformed. When the amount of solvent is large and the relative amount of dissolving 4-(1-((4-methylbenzothiophen-3-yl)methyl)benzimidazol-2-ylthio)butanoic acid sulfate is high, even if all the suspended crystals have been transformed to the crystal-SC-form, crystals in a form other than crystal-SC may be crystallized during the course of cooling to a temperature for filtration. Crystal-SC can be crystallized, however, by decreasing the cooling speed.

Crystal-SC of the sulfate can be obtained by appropriately selecting the transformation temperature, solvent amount, and cooling speed in combination, considering the above points. Stirring is preferred since it accelerates the transformation rate.

All of the hydrochloride and crystal-HA, crystal-HB, and crystal-HC thereof can be manufactured by the above methods. The solvent to be used is similar to that for the sulfate. Crystal-HA can be crystallized by cooling a 1,4-dioxane solution of the hydrochloride without seeding. Crystal-HB can be crystallized by cooling a solution in a solvent such as 4-methyl-2-pentanone, butyl acetate, a mixed solvent of acetic acid and 2-butanone, a mixed solvent of acetic acid and butyl acetate, a mixed solvent of dimethylformamide and 2-butanone, or a mixed solvent of dimethylformamide and butyl acetate, without seeding. Crystal-HC can be crystallized by cooling an acetic acid solution without seeding.

Crystal-HB of the hydrochloride can be obtained by suspending crystal-HA, crystal-HC, or a mixture thereof in a solvent such as 2-butanone, 1,4-dioxane, or a like to perform solvent-mediated transformation. It is effective to add crystal-HB as seed crystals in order to reduce the time required for solvent-mediated transformation.

All the methanesulfonic acid and crystal-MA, crystal-MB, crystal-MC, crystal-MD, and crystal-ME thereof can be manufactured by the above methods. The solvent to be used is similar to that for the sulfate. Crystal-MA can be crystallized by cooling a solution in a mixed solvent of acetic acid and 2-butanone without seeding. Crystal-MB can be crystallized by cooling an acetic acid solution without seeding. Crystal-MC can be crystallized by cooling a solution in 4-methyl-2-pentanone. Crystal-MD can be crystallized by cooling a solution in butyl acetate. Crystal-ME can be crystallized by cooling a solution in 1,4-dioxane.

Crystal-MA of the methanesulfonic acid can be obtained by suspending crystal-MB, crystal-MC, crystal-MD, crystal-ME, or a mixture of crystals in two or more forms selected from these in 2-butanone to perform solvent-mediated transformation. It is effective to add crystal-MA as seed crystals in order to reduce the time required for solvent-mediated transformation. Crystal-ME can be obtained by suspending crystal-MA, crystal-MB, crystal-MC, crystal-MD, or a mixture of crystals in two or more forms selected from these in 2-butanone to perform solvent-mediated transformation. It is effective to add crystal-ME as seed crystals in order to reduce the time required for solvent-mediated transformation.

The crystals may be collected by an ordinary method such as filtration, pressurized filtration, suction filtration, and centrifugation; and may be dried using an ordinary method such as air drying, drying under reduced pressure, heat drying, and heat drying under reduced pressure.

When a mixture of two or more forms of crystals is desired, the mixture can be obtained not only by mixing the individual forms of crystals that have been produced separately but also by producing the mixture at a time. In order to obtain a mixture at a predetermined ratio, however, the conditions must be determined based on detailed preliminary examination. The composition ratio may be quantified based on analytical methods such as powder X-ray diffractometry, infrared absorption spectroscopy, and thermal analysis, although the validity of such methods depends on the combination and ratio of crystal forms. In this case, the solvent-mediated transformation is a relatively easy production method, since in-time monitoring of the composition ratio may be performed.

Each of the crystal forms according to the present invention can be distinguished from other crystal forms by its characteristic powder X-ray diffraction pattern and infrared absorption spectrum, but the contamination ratio of other crystal forms is not specifically limited. When crystals in a particular crystal form are to be obtained alone, contamination in such a level that presence of another crystal form cannot be detected by these pattern or spectrum is allowable. When the crystal in each particular form is used as a drug substance of a pharmaceutical, it does not necessarily mean that incorporation of crystals in another form is unallowable.

All the acid salts or the crystals thereof according to the present invention can be used alone as an active ingredient of a pharmaceutical. Further, a mixture of two or more acid salts or the crystals thereof according to the present invention can be also used as an active ingredient of a pharmaceutical.

In the present invention, acid salt, such as sulfate, hydrochloride, and methanesulfonate, of 4-(1-((4-methylbenzothiophen-3-yl)methyl)benzimidazol-2-ylthio)butanoic acid obtained as crystals is advantageous in terms of handleability, reproducibility, and stability in manufacturing and storage stability as compared with the same salt that is not in a crystalline state.

For the sulfate, crystal-SC and crystal-SF are particularly excellent and preferably used. Since they are crystals, crystal-SA and crystal-SB are also easy to handle, effectively purified and dried, and stable in storage. Also, these crystal forms as well as crystal-SF are useful as source materials (manufacturing intermediates) for transformation to crystal-SC. In addition, crystal-SG is particularly preferable as a source material for transformation to crystal-SC, since transformation from crystal-SG to crystal-SC proceeds very rapidly.

For the hydrochloride, crystal-HB is preferably used because of the stability. Crystal-HA and crystal-HC also have good handleability and high storage stability since they are crystals, and they are useful as source materials (manufacturing intermediates) for transformation to crystal-HB.

For the methanesulfonate, crystal-MA and crystal-ME are relatively stable and preferably used. Crystal-MB, crystal-MC, and crystal-MD also have good handleability as crystals, and they are useful as source materials (manufacturing intermediates) for transformation to crystal-MA or crystal-ME.

In the present invention, all of the acid salt such as sulfate, hydrochloride, and methanesulfonate, of 4-(1-((4-methylbenzothiophen-3-yl)methyl)benzimidazol-2-ylthio)butanoic acid and the crystals thereof are more soluble in aqueous solvents than 4-(1-((4-methylbenzothiophen-3-yl)methyl)benzimidazol-2-ylthio)butanoic acid and its crystal. For example, when the sulfate in a crystalline state is used as a drug substance of a pharmaceutical composition, the absorption increases by 10 to 30%, preferably 15 to 25%, so that the dose can be decreased.

A formulation comprising the compound of the present invention as an active ingredient can be prepared using carriers, excipients, and other additives commonly used for formulation. The carriers and excipients for formulation may be either solid or liquid and include, for example, lactose, magnesium stearate, starch, talc, gelatin, agar, pectin, Arabic gum, olive oil, sesame oil, cocoa butter, ethylene glycol, and other substances routinely used. Administration may be conducted orally using tablets, pills, capsules, granules, powder, liquid, or the like, or parenterally using injections such as intravenous injections and intramuscular injections, suppositories, percutaneous administration, or the like.

In administration of the acid salt, such as sulfate, hydrochloride, and methanesulfonate, of 4-(1-((4-methylbenzothiophen-3-yl)methyl)benzimidazol-2-ylthio)butanoic acid or the crystals thereof according to the present invention, the dose is dependent on the disease, administration route, symptoms, age, sex, body weight of a patient, and others, but generally approximately 1 to 500 mg/day/person, and preferably 10 to 300 mg/day/person for oral administration. The dose is approximately 0.1 to 100 mg/day/person, preferably 0.3 to 30mg /day/person for parenteral administration such as intravenous, subcutaneous, intramuscular, percutaneous, rectal, nasal, eye drop, inhalation or the like.

When the acid salt, such as sulfate, hydrochloride, and methanesulfonate, of 4-(1-((4-methylbenzothiophen-3-yl)methyl)benzimidazol-2-ylthio)butanoic acid or the crystals thereof according to the present invention are used as a preventive and/or therapeutic agent, they may be administered depending on the symptoms in accordance with previously known methods.

Target diseases of the preventive and/or therapeutic agent according to the present invention include respiratory diseases such as bronchial asthma; inflammatory/allergic diseases such as allergic rhinitis, atopic dermatitis, and urticaria; circulatory diseases such as sclerotic vascular lesion, intravascular stenosis, peripheral circulatory disturbance, renal insufficiency, and cardiac insufficiency; and bone/cartilage metabolic diseases such as rheumatoid and osteoarthritis.

Since the acid salt such as sulfate, hydrochloride, and methanesulfonate of 4-(1-((4-methylbenzothiophen-3-yl)methyl)benzimidazol-2-ylthio)butanoic acid and the crystals thereof according to the present invention are highly soluble in an aqueous solvent and a pharmaceutical composition comprising the same is excellent in absorption, enabling the reduction of dose.

Use of these crystals is expected to be advantageous in terms of storage stability of a pharmaceutical composition and a drug substance, control of manufacturing processes, and others.

### Examples

Hereinafter will be described methods for preparing sulfate, hydrochloride, and methanesulfonate of 4-(1-((4-methylbenzothiophen-3-yl)methyl)benzimidazol-2-ylthio)butanoic acid and crystals thereof according to the present invention, referring to Examples. However, the present invention is not limited by these Examples.

The crystals according to the present invention were analyzed under the following conditions.
Measurement conditions for powder X-ray diffraction pattern
Apparatus: RIGAKU ROTAFLEX RU300 (Powder X-ray diffractometer)
X-ray source: Cu-Kα (λ = 1.5418 Å), 50 kV-200 mA
Slit: DS1°-SS1°-RS 0.15 mm - graphite monochromator - 0.45 mm
Method: 2θ-θ scan, 0.05 step/sec, scan range 5 to 80°
Measurement conditions for infrared absorption spectrum
Apparatus: Shimadzu FTIR-8000
Infrared absorption spectra were recorded in potassium bromide disc with FT-IR (Resolution: 4 cm⁻¹, accumulation number: 40, GAIN: AUTO).

### [Example 1]

### Manufacturing of 4-(1-((4-methylbenzothiophen-3-yl)methyl)benzimidazol-2-ylthio)butanoic acid sulfate

In accordance with the method described in Example 2 of Patent Document 4, 10 g of 4-(1-((4-methylbenzothiophen-3-yl)methyl)benzimidazol-2-ylthio)butanoic acid was obtained. The compound was identified based on the molecular weight with LC-MS. In 10 mL of dimethylsulfoxide-d₆ (NMR solvent), 0.1 g of 4-(1-((4-methylbenzothiophen-3-yl)methyl)benzimidazol-2-ylthio)butanoic acid was dissolved, and 0.025 g of sulfuric acid was added to the solution to obtain a solution of 4-(1-((4-methylbenzothiophen-3-yl)methyl)benzimidazol-2-ylthio)butanoic acid sulfate. The NMR spectrum was measured to confirm the structure of compound.
¹H-NMR (200 MHz, DMSO-d₆, TMS reference, δ): 1.88-2.05 (m, 2H), 2.36 (t, J = 7.2 Hz, 2H), 2.87 (s, 3H), 3.44 (t, J = 7.2 Hz, 2H), 6.00 (s, 2H), 6.61 (s, 1H), 7.2-7.4 (m, 4H), 7.66-7.84 (m, 3H), 11.0 (br, 3H)

### [Example 2]

### Manufacturing of crystal-SF of 4-(1-((4-methylbenzothiophen-3-yl)methyl)benzimidazol-2-ylthio)butanoic acid sulfate

Eight grams of 4-(1-((4-methylbenzothiophen-3-yl)methyl)benzimidazol-2-ylthio)butanoic acid were dissolved in 80 mL of tetrahydrofuran while heated to reflux in an oil bath. Here was added 10 mL of a tetrahydrofuran solution containing 2.2 g of sulfuric acid, and the mixture was filtered in hot to remove insoluble matters. The solution was again heated to reflux to ensure dissolution and cooled to 20°C in an oil bath with stirring for crystallization. The crystals were collected by filtration and dried at 60°C under reduced pressure for 4 hours. The obtained crystals were identified as crystal-SF by XRD and IR. Yield: 81 %.
¹H-NMR (200 MHz, DMSO-d₆, TMS reference, δ) 1.88-2.05 (m, 2H), 2.36 (t, J = 7.2 Hz, 2H), 2.87 (s, 3H), 3.44 (t, J = 7.2 Hz, 2H), 6.00 (s, 2H), 6.61 (s, 1H), 7.2-7.4 (m, 4H), 7.66-7.84 (m, 3H), 11.0 (br, 3H)
EA (obs, %) C: 50.77, H: 4.60, N: 5.81, S: 19.54; EA (cal, %) C: 51.00, H: 4.48, N: 5.66, S: 19.45

The chymase inhibitory activity of 4-(1-((4-methylbenzothiophen-3-yl)methyl)benzimidazol-2-ylthio)butanoic acid sulfate obtained was measured in accordance with the inhibition assay against enzymatic activity of recombinant human mast cell chymase described in Example 17 of Patent Document 4. Specifically, a recombinant pro-type human mast cell chymase was prepared by the method of Urata et al. (Journal of Biological Chemistry 266, 17173 (1991)). That is, the enzyme was purified using Heparin Sepharose (Pharmacia) from a culture supernatant of insect cells (Th5) infected with recombinant baculovirus carrying cDNA encoding human mast cell chymase. The chymase was activated by the method of Murakami et al. (Journal of Biological Chemistry 270, 2218 (1995)) and purified with Heparin Sepharose to obtain activated human mast cell chymase. To 50 µl of buffer A (0.5 to 3.0 M NaCl, 50 mM Tris-HCl, pH 8.0) containing 1 to 5 ng of activated human mast cell chymase thus obtained, was added 2 µl of a DMSO solution containing the compound according to the present invention, 4-(1-((4-methylbenzothiophen-3-yl)methyl)benzimidazol-2-ylthio)butanoic acid sulfate. Subsequently here was added 50 µl of buffer A containing 0.5 mM of succinyl-alanyl-histidyl-prolyl-phenylalanyl-p-nitroanilide (Backem Holding AG) as a substrate, and the mixture was incubated at room temperature for 5 minutes. The variation of absorbance at 405 nm with time was measured to determine the inhibitory activity.

The results showed that the sulfate of the present invention had an inhibitory activity represented by IC₅₀ of 1 nM or more and 10 nM or less.

### [Example 3]

### Manufacturing of crystal-SF of 4-(1-((4-methylbenzothiophen-3-yl)methyl)benzimidazol-2-ylthio)butanoic acid sulfate

Ten grams of 4-(1-((4-methylbenzothiophen-3-yl)methyl)benzimidazol-2-ylthio)butanoic acid were dissolved in 30 mL of acetic acid while heated in an oil bath so that the temperature of solution was 90°C. To the resultant solution, 8 mL of an acetic acid solution containing 2.7 g of sulfuric acid was added, and the mixture was filtered in hot to remove insoluble matters. After dissolution was confirmed at the solution temperature of 90°C, the solution was cooled to 20°C in an oil bath with stirring for crystallization. The crystals were collected by filtration and dried at 60°C under reduced pressure for 8 hours. The obtained crystals were identified as crystal-SF by XRD and IR. Yield: 54%.

### [Example 4]

### Manufacturing of crystal-SG of 4-(1-((4-methylbenzothiophen-3-yl)methyl)benzimidazol-2-ylthio)butanoic acid sulfate

Four grams of 4-(1-((4-methylbenzothiophen-3-yl)methyl)benzimidazol-2-ylthio)butanoic acid were dissolved in 12 mL of acetic acid while heated in an oil bath so that the temperature of solution was 90°C. To the resultant solution, 1.1 g of sulfuric acid was added, and the solution was filtered in hot to remove insoluble matters. When the temperature of solution was 75°C, dissolution was confirmed and here was added 0.08 g of crystal-SC of 4-(1-((4-methylbenzothiophen-3-yl)methyl)benzimidazol-2-ylthio)butanoic acid sulfate. The mixture was stirred at the same temperature for 5 hours for crystallization. The crystals were collected by filtration at the same temperature and dried at 60°C under reduced pressure for 4 hours. The obtained crystals were identified as crystal-SG by XRD and IR. Yield: 25%.

### [Example 5]

### Manufacturing of crystal-SG of 4-(1-((4-methylbenzothiophen-3-yl)methyl)benzimidazol-2-ylthio)butanoic acid sulfate

Four grams of 4-(1-((4-methylbenzothiophen-3-yl)methyl)benzimidazol-2-ylthio)butanoic acid were dissolved in 12 mL of acetic acid while heated in an oil bath so that the temperature of solution was 90°C. To the resultant solution, 1.1 g of sulfuric acid was added and the mixture was filtered in hot to remove insoluble matters. When the temperature of solution was 75°C, dissolution was confirmed, here was added 0.08 g of crystal-SC of 4-(1-((4-methylbenzothiophen-3-yl)methyl)benzimidazol-2-ylthio)butanoic acid sulfate, and quickly, 120 mL of heptane was added to crystallize. The mixture was cooled to 20°C with stirring, and the crystals were collected by filtration at the same temperature and dried at 60°C under reduced pressure for 4 hours. The obtained crystals were identified as crystal-SG by XRD and IR. Yield: 83%.

### [Example 6]

### Manufacturing of crystal-SA of 4-(1-((4-methylbenzothiophen-3-yl)methyl)benzimidazol-2-ylthio)butanoic acid sulfate

Ten grams of 4-(1-((4-methylbenzothiophen-3-yl)methyl)benzimidazol-2-ylthio)butanoic acid were dissolved in 30 mL of acetic acid while heated in an oil bath so that the temperature of solution was 90°C. To the resultant solution, 2.7 g of sulfuric acid was added and the mixture was filtered in hot to remove insoluble matters. After dissolution was confirmed when the solution temperature was 90°C, here was added 100 mL of 2-butanone, and the mixture was cooled to 20°C with stirring in an oil bath for crystallization. The crystals were collected by filtration and dried at 60°C under reduced pressure for 4 hours. The obtained crystals were identified as crystal-SA by XRD and IR. Yield: 80%.

### [Example 7]

### Manufacturing of crystal-SB of 4-(1-((4-methylbenzothiophen-3-yl)methyl)benzimidazol-2-methyl)butanoic acid sulfate

Eight grams of 4-(1-((4-methylbenzothiophen-3-yl)methyl)benzimidazol-2-ylthio)butanoic acid were dissolved in 80 mL of acetic acid while heated in an oil bath so that the temperature of solution was 90°C. To the resultant solution, 2.2 g of sulfuric acid was added and the mixture was filtered in hot to remove insoluble matters. After dissolution was confirmed when the solution temperature was 90°C, here was added 40 mL of 2-butanone and the mixture was cooled to 20°C with stirring in an oil bath for crystallization. The crystals were collected by filtration and dried at 60°C under reduced pressure for 4 hours. The obtained crystals were identified as crystal-SB by XRD and IR. Yield: 60%.

### [Example 8]

### Manufacturing of crystal-SC of 4-(1-((4-methylbenzothiophen-3-yl)methyl)benzimidazol-2-ylthio)butanoic acid sulfate

A mixture of 90 g of crystal-SF and 10 g of crystal-SA of 4-(1-((4-methylbenzothiophen-3-yl)methyl)benzimidazol-2-ylthio)butanoic acid sulfate was suspended in 100 mL of2-butanone. This mixture was heated to reflux in an oil bath with stirring for 24 hours and then cooled to 20°C. The crystals were collected by filtration and dried at 60°C under reduced pressure for 4 hours. The obtained crystals were identified as crystal-SC by XRD and IR. Yield: 90%.

### [Example 9]

### Solubility of 4-(1-((4-methylbenzothiophen-3-yl)methyl)benzimidazol-2-ylthio)butanoic acid sulfate

Table 14 shows the solubilities of each crystal form of 4-(1-((4-methylbenzothiophen-3-yl)methyl)benzimidazol-2-ylthio)butanoic acid sulfate in Solution 1 and Solution 2 of the Japanese Pharmacopoeia and water. In accordance with the General Rules of the Japanese Pharmacopoeia 14th Edition, solubility (µg/mL) herein refers to an amount of a substance dissolved in a solvent after the substance in powder is placed in the solvent and the sample is shaken strongly for 30 seconds every 5 minutes at 20±5°C within 30 minutes.

For comparison, the solubility of Crystal A of 4-(1-((4-methylbenzothiophen-3-yl)methyl)benzimidazol-2-ylthio)butanoic acid, which is not a salt, is shown in Table 14. This crystalline sample was manufactured by the following method.

To 10 g of 4-(1-((4-methylbenzothiophen-3-yl)methyl)benzimidazol-2-ylthio)butanoic acid, 50 mL of butyl acetate was added, and the mixture was heated to reflux in an oil bath and then filtered in hot to remove insoluble matters. The filtrate was again heated to reflux to ensure dissolution and cooled at a rate of approximately 40°C/hour with stirring in an oil bath. Crystallization started when the solution temperature was approximately 90°C. The mixture was cooled to 20°C and the crystals were collected by filtration and dried under reduced pressure at 60°C for 4 hours. Yield 75%.

**Table 14**

| | Sulfate Crystal-SA | Sulfate Crystal-SB | Sulfate Crystal-SC | Sulfate Crystal-SF | Free base Crystal A |
|---|---|---|---|---|---|
| Water | 0.0 | 0.8 | 0.1 | 0.1 | 0.0 |
| Solution 1 | 8.6 | 19.8 | 10.6 | 7.6 | 2.5 |
| Solution 2 | 67.3 | 24.2 | 72.4 | 74.0 | 1.9 |

### [Example 10]

### Manufacturing of 4-(1-((4-methylbenzothiophen-3-yl)methyl)benzimidazol-2-ylthio)butanoic acid hydrochloride

In accordance with the method described in Example 2 of Patent Document 4, 10 g of 4-(1-((4-methylbenzothiophen-3-yl)methyl)benzimidazol-2-ylthio)butanoic acid was obtained. The 0.1 g of 4-(1-((4-methylbenzothiophen-3-yl)methyl)benzimidazol-2-ylthio)butanoic acid was dissolved in 10 mL of dimethylsulfoxide-d₆ (NMR solvent). And this solution was gently bubbled with approximately 20 mL of hydrogen chloride gas to obtain a solution of 4-(1-((4-methylbenzothiophen-3-yl)methyl)benzimidazol-2-ylthio)butanoic acid hydrochloride. The product was identified by NMR.
¹H-NMR (400 MHz, DMSO-d₆, TMS reference, δ) 1.96-1.99 (m, 2H), 2.40 (t, J = 7.2 Hz, 2H), 2.88 (s, 3H), 3.57 (t, J = 7.2 Hz, 2H), 6.03 (s, 2H), 6.70 (s, 1H), 7.27 (d, J = 7.2 Hz, 1H), 7.30 (t, J = 7.2 Hz, 1H), 7.37-7.47 (m, 2H), 7.75 (d, J = 8.0 Hz, 1H), 7.81 (d, J = 8.0 Hz, 1H)

### [Example 11]

### Manufacturing of crystal-HB of 4-(1-((4-methylbenzothiophen-3-yl)methyl)benzimidazol-2-ylthio)butanoic acid hydrochloride

To 10g of 4-(1-((4-methylbenzothiophen-3-yl)methyl)benzimidazol-2-ylthio)butanoic acid, 60 mL of acetic acid and 60 mL of 2-butanone were added. The solid was dissolved while the mixture was heated to reflux in an oil bath. To the resultant solution, 3 mL of concentrated hydrochloric acid was added, and the solution was filtered in hot to remove insoluble matters. The filtrate was again heated to reflux to ensure dissolution and cooled to 20°C with stirring in an oil bath for crystallization. The crystals were collected by filtration and dried at 60°C under reduced pressure for 5 hours. The obtained crystals were identified crystal-HB by XRD and IR. Yield: 92%.
¹H-NMR (400MHz, CDCl₃/DMSO-d₆, TMS reference, δ) 2.11-2.15 (m, 2H), 2.53 (t, J = 7.0 Hz, 2H), 2.89 (s, 3H), 3.85 (t, J = 7.4 Hz,2H), 6.03 (s, 2H), 6.52 (s, 1H), 7.23 (d, J = 7.2Hz, 1H), 7.31 (d, J = 7.6 Hz, 1H), 7.45-7.56 (m, 3H), 7.73 (d, J = 8.0 Hz, 1H), 8.02 (d, J = 8.0 Hz, 1H)

Then, the chymase inhibitory activity of 4-(1-((4-methylbenzothiophen-3-yl)methyl)benzimidazol-2-ylthio)butanoic acid hydrochloride obtained was measured by inhibition assay against enzymatic activity of recombinant human mast cell chymase by the method similar to that in Example 2.

The results showed that the hydrochloride of the present invention had inhibitory activity represented by IC₅₀ of 1 nM or more and 10 nM or less.

### [Example 12]

### Manufacturing of crystal-HA of 4-(1-((4-methylbenzothiophen-3-yl)methyl)benzimidazol-2-ylthio)butanoic acid hydrochloride

One gram of 4-(1-((4-methylbenzothiophen-3-yl)methyl)benzimidazol-2-ylthio)butanoic acid was dissolved in 180 mL of 1,4-dioxane while the mixture was heated to reflux in an oil bath. To the resultant solution, 1 mL of a 1,4-dioxane solution of hydrogen chloride (4 mol/L) was added and the solution was filtered in hot to remove insoluble matters. The filtrate was again heated to reflux to ensure dissolution and cooled to 20°C with stirring in an oil bath for crystallization. The crystals were collected by filtration and dried at 60°C under reduced pressure for 5 hours. The obtained crystals were identified as crystal-HA by XRD and IR. Yield: 99%.

### [Example 13]

### Manufacturing of crystal-HC of 4-(1-((4-methylbenzothiophen-3-yl)methyl)benzimidazol-2-ylthio)butanoic acid hydrochloride

Eight grams of 4-(1-((4-methylbenzothiophen-3-yl)methyl)benzimidazol-2-ylthio)butanoic acid were dissolved in 120 mL of acetic acid while the mixture was heated in an oil bath. To the resultant solution, 6 mL of a 1,4-dioxane solution of hydrogen chloride (4 mol/L) was added and the solution was filtered in hot to remove insoluble matters. The filtrate was again heated to ensure dissolution and cooled to 20°C with stirring in an oil bath for crystallization. The crystals were collected by filtration and dried at 60°C under reduced pressure for 5 hours. The obtained crystals were identified as crystal-HC by XRD and IR. Yield: 85%.

### [Example 14]

### Manufacturing of crvstal-HB of 4-(1-((4-methylbenzothiophen-3-yl)methyl)benzimidazol-2-ylthio)butanoic acid hydrochloride

A mixture of 10 g of crystal-HA and 1 g of crystal-HB of 4-(1-((4-methylbenzothiophen-3-yl)methyl)benzimidazol-2-ylthio)butanoic acid hydrochloride were suspended in 50 mL of 2-butanone. The mixture was heated to reflux with stirring in an oil bath for 48 hours and then cooled to 20°C. The crystals were collected by filtration and dried at 60°C under reduced pressure for 4 hours. The obtained crystals were identified as crystal-HB by XRD and IR. Yield: 96%.

### [Example 15]

### Manufacturing of 4-(1-((4-methylbenzothiophen-3-yl)methyl)benzimidazol-2-ylthio)butanoic acid methanesulfonate

In accordance with the method in Example 2 of Patent Document 4, 10 g of 4-(1-((4-methylbenzothiophen-3-yl)methyl)benzimidazol-2-ylthio)butanoic acid, was obtained. The 0.1g of 4-(1-((4-methylbenzothiophen-3-yl)methyl)benzimidazol-2-ylthio)butanoic acid was dissolved in 10 mL of dimethylsulfoxide-d₆ (NMR solvent). To this solution, 0.024 g of methanesulfonic acid was added to obtain a solution of 4-(1-((4-methylbenzothiophen-3-yl)methyl)benzimidazol-2-ylthio)butanoic acid methanesulfonate. The product was identified by NMR.
¹H-NMR (400MHz, DMSO-d₆, TMS reference, δ) 1.94-1.98 (m, 2H), 2.36 (t, J = 7.2 Hz, 2H), 2.42 (s, 3H), 2.88 (s, 3H), 3.47 (t, J = 7.2 H z,2H), 6.03 (s, 2H), 6.68 (s, 1H), 7.23 (d, J = 7.2 Hz, 1H), 7.30 (t, J = 7.2 Hz,1H), 7.38-7.50 (m, 2H), 7.74 (d, J = 8.0 Hz, 1H), 7.81 (d, J = 8.0 Hz, 1H)

### [Example 16]

### Manufacturing of crystal-MA of 4-(1-((4-methylbenzothiophen-3-yl)methyl)benzimidazol-2-ylthio)butanoic acid methanesulfonate

To 20 g of 4-(1-((4-methylbenzothiophen-3-yl)methyl)benzimidazol-2-ylthio)butanoic acid, 120 mL of acetic acid and 120 mL of 2-butanone were added, and the solid was dissolved while the mixture was heated to reflux in an oil bath. To the resultant solution, 5 g of methanesulfonic acid was added and the solution was filtered in hot to remove insoluble matters. The filtrate was again heated to reflux to ensure dissolution and cooled to 20°C with stirring in an oil bath for crystallization. The crystals were collected by filtration and dried at 60°C under reduced pressure for 5 hours. The obtained crystals were identified as crystal-MA by XRD and IR. Yield: 88%.
¹H-NMR (400 MHz, CDCl₃/DMSO-d₆, TMS reference, δ) 2.07-2.13 (m, 2H), 2.47 (t, J = 7.3 Hz, 2H), 2.77 (s, 3H), 2.90 (s, 3H), 3.85 (t, J = 7.4 Hz,2H), 6.05 (s, 2H), 6.55 (s, 1H), 7.22 (d, J = 7.2 Hz, 1H), 7.30 (d, J = 7.6 Hz, 1H), 7.44-7.51 (m, 2H), 7.61 (d, J = 8.4 Hz, 1H), 7.72 (d, J = 8.0 Hz, 1H), 8.02 (d, J = 8.4 Hz, 1H)

### [Example 17]

### Manufacturing of crystal-MB of 4-(1-((4-methylbenzothiophen-3-yl)methyl)benzimidazol-2-ylthio)butanoic acid methanesulfonate

Ten grams of 4-(1-((4-methylbenzothiophen-3-yl)methyl)benzimidazol-2-ylthio)butanoic acid were dissolved in 60 mL of acetic acid while heated in an oil bath. To the resultant solution, 3 g of methanesulfonic acid was added and the solution was filtered in hot to remove insoluble matters. The filtrate was again heated to reflux to ensure dissociation and cooled to 20°C with stirring in an oil bath for crystallization. The crystals were collected by filtration and dried at 60°C under reduced pressure for 8 hours. The obtained crystals were identified as crystal-MB by XRD and IR. Yield: 76%.

### [Example 18]

### Manufacturing of crystal-MC of 4-(1-((4-methylbenzothiophen-3-yl)methyl)benzimidazol-2-ylthio)butanoic acid methanesulfonate

One gram of 4-(1-((4-methylbenzothiophen-3-yl)methyl)benzimidazol-2-ylthio)butanoic acid was dissolved in 100 mL of 4-methyl-2-pentanone while the mixture was heated to reflux in an oil bath. To the resultant solution, 0.3 g of methanesulfonic acid was added and the solution was filtered in hot to remove insoluble matters. The filtrate was again heated to reflux to ensure dissolution and cooled to 20°C with stirring in an oil bath for crystallization. The crystals were collected by filtration and dried at 60°C under reduced pressure for 4 hours. The obtained crystals were identified as crystal-MC by XRD and IR. Yield: 93%.

### [Example 19]

### Manufacturing of crystal-MD of 4-(1-((4-methylbenzothiophen-3-yl)methyl)benzimidazol-2-ylthio)butanoic acid methanesulfonate

One gram of 4-(1-((4-methylbenzothiophen-3-yl)methyl)benzimidazol-2-ylthio)butanoic acid was dissolved in 300 mL butyl acetate while the mixture was heated to reflux in an oil bath. To the resultant solution, 0.3 g of methanesulfonic acid was added and the solution was filtered in hot to remove insoluble matters. The filtrate was again heated to reflux to ensure dissolution and cooled to 20°C with stirring in an oil bath for crystallization. The crystals were collected by filtration and dried at 60°C under reduced pressure for 6 hours. The obtained crystals were identified as crystal-MD by XRD and IR. Yield: 90%.

### [Example 20]

### Manufacturing of crystal-ME of 4-(1-((4-methylbenzothiophen-3-yl)methyl)benzimidazol-2-ylthio)butanoic acid methanesulfonate

Five grams of 4-(1-((4-methylbenzothiophen-3-yl)methyl)benzimidazol-2-ylthio)butanoic acid were dissolved in 350 mL of 1,4-dioxane while heated in an oil bath. To the resultant solution, 1.5 g of methanesulfonic acid was added and the solution was filtered in hot to remove insoluble matters. The filtrate was again heated to reflux to ensure dissolution and cooled to 20°C with stirring in an oil bath for crystallization. The crystals were collected by filtration and dried at 60°C under reduced pressure for 8 hours. The obtained crystals were identified as crystal-ME by XRD and IR. Yield: 88%.

### [Example 21]

### Manufacturing of crystal-MA of 4-(1-((4-methylbenzothiophen-3-yl)methyl)benzimidazol-2-ylthio)butanoic acid methanesulfonate

A mixture of 19 g of crystal-MA and 1 g of crystal-MB of 4-(1-((4-methylbenzothiophen-3-yl)methyl)benzimidazol-2-ylthio)butanoic acid methanesulfonate were suspended in 100 mL of 2-butanone, and the resultant mixture was heated to reflux with stirring in an oil bath for 24 hours. The mixture was cooled to 20°C and the crystals were collected by filtration and dried at 60°C under reduced pressure for 4 hours. The obtained crystals were identified as crystal-MA by XRD and IR. Yield: 94%.

### Industrial Applicability

The acid salts such as sulfate, hydrochloride, and methanesulfonate, of 4-(1-((4-methylbenzothiophen-3-yl)methyl)benzimidazol-2-ylthio)butanoic acid according to the present invention and crystals thereof have an in vivo chymase inhibitory activity and can be used as a preventive or therapeutic agent for inflammatory diseases, allergic diseases, respiratory diseases, circulatory diseases, or bone/cartilage metabolic diseases.

The acid salts of benzimidazole derivative according to the present invention and the crystals thereof are highly soluble, and a pharmaceutical composition comprising the same is excellent in absorption property. The crystals are expected to be advantageous in terms of storage stability of a pharmaceutical composition and a drug substance, control of manufacturing processes thereof, and others. The crystals can also be used as an intermediate of a drug substance.

## Claims

1. An acid salt of 4-(1-((4-methylbenzothiophen-3-yl)methyl)benzimidazol-2-ylthio)butanoic acid.

2. A crystal of an acid salt of 4-(1-((4-methylbenzothiophen-3-yl)methyl)benzimidazol-2-ylthio)butanoic acid.

3. The salt according to claim 1 wherein the acid salt of 4-(1-((4-methylbenzothiophen-3-yl)methyl)benzimidazol-2-ylthio)butanoic acid is sulfate, hydrochloride, or methanesulfonate.

4. The crystal according to claim 2 wherein the acid salt of 4-(1-((4-methylbenzothiophen-3-yl)methyl)benzimidazol-2-ylthio)butanoic acid is sulfate, hydrochloride, or methanesulfonate.

5. A crystal of 4-(1-((4-methylbenzothiophen-3-yl)methyl)benzimidazol-2-ylthio)butanoic acid sulfate represented by formula (I) below:

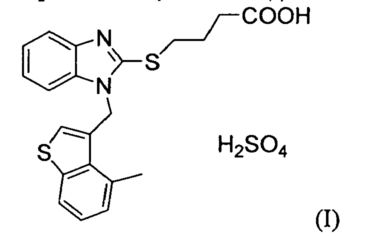

6. The crystal of the sulfate according to claim 5 showing a powder X-ray diffraction pattern with characteristic peaks at diffraction angles 2θ of approximately 5.3°, 11.7°, 15.9°, 21.9°, 23.1 °, and 27.5° (Crystal-SA).

7. The crystal of the sulfate according to claim 5 showing a powder X-ray diffraction pattern approximately the same as that shown in Figure 1 (Crystal-SA).

8. The crystal of the sulfate according to claim 5 showing a powder X-ray diffraction pattern with characteristic peaks at diffraction angles 2θ of approximately 11.4°, 13.8°, 15.9°, 16.6°, and 22.8° (Crystal-SB).

9. The crystal of the sulfate according to claim 5 showing a powder X-ray diffraction pattern approximately the same as that shown in Figure 2 (Crystal-SB).

10. The crystal of the sulfate according to claim 5 showing a powder X-ray diffraction pattern with characteristic peaks at diffraction angles 2θ of approximately 8.1°, 13.5°, 22.0°, 22.8°, and 24.2° (Crystal-SC).

11. The crystal of the sulfate according to claim 5 showing a powder X-ray diffraction pattern approximately the same as that shown in Figure 3 (Crystal-SC).

12. The crystal of the sulfate according to claim 5 showing a powder X-ray diffraction pattern with characteristic peaks at diffraction angles 2θ of approximately 12.9°, 21.2°, 22.9°, 24.7°, and 27.3° (Crystal-SF).

13. The crystal of the sulfate according to claim 5 showing a powder X-ray diffraction pattern approximately the same as that shown in Figure 4 (Crystal-SF).

14. The crystal of the sulfate according to claim 5 showing a powder X-ray diffraction pattern with characteristic peaks at diffraction angles 2θ of approximately 12.2°, 13.5°, 18.5°, 22.4°, and 23.7° (Crystal-SG).

15. The crystal of the sulfate according to claim 5 showing a powder X-ray diffraction pattern approximately the same as that shown in Figure 5 (Crystal-SG).

16. The crystal of the sulfate according to claim 5 whose infrared absorption spectrum in potassium bromide exhibits peaks at wavenumbers of approximately 1715, 1456, 1203, 1067, 880, and 756 cm⁻¹ (Crystal-SA).

17. The crystal of the sulfate according to claim 5 whose infrared absorption spectrum in potassium bromide exhibits the absorption pattern shown in Figure 6 (Crystal-SA).

18. The crystal of the sulfate according to claim 5 whose infrared absorption spectrum in potassium bromide exhibits peaks at wavenumbers of approximately 1709, 1468, 1230, 1163, 1063, 862, and 754 cm⁻¹ (Crystal-SB).

19. The crystal of the sulfate according to claim 5 whose infrared absorption spectrum in potassium bromide exhibits the absorption pattern shown in Figure 7 (Crystal-SB).

20. The crystal of the sulfate according to claim 5 whose infrared absorption spectrum in potassium bromide exhibits peaks at wavenumbers of approximately 1705, 1459, 1325, 1238, 1152, 1065, 874, and 762 cm⁻¹ (Crystal-SC).

21. The crystal of the sulfate according to claim 5 whose infrared absorption spectrum in potassium bromide exhibits the absorption pattern shown in Figure 8 (Crystal-SC).

22. The crystal of the sulfate according to claim 5 whose infrared absorption spectrum in potassium bromide exhibits peaks at wavenumbers of approximately 1726, 1459, 1316, 1248, 1154, 1046, 869, and 768 cm⁻¹ (Crystal-SF).

23. The crystal of the sulfate according to claim 5 whose infrared absorption spectrum in potassium bromide exhibits the absorption pattern shown in Figure 9 (Crystal-SF).

24. The crystal of the sulfate according to claim 5 whose infrared absorption spectrum in potassium bromide exhibits peaks at wavenumbers of approximately 1709, 1467, 1317, 1234, 1152, 1065, 872, and 761 cm⁻¹ (Crystal-SG).

25. The crystal of the sulfate according to claim 5 whose infrared absorption spectrum in potassium bromide exhibits the absorption pattern shown in Figure 10 (Crystal-SG).

26. A crystal of 4-(1-((4-methylbenzothiophen-3-yl)methyl)benzimidazol-2-ylthio)butanoic acid hydrochloride represented by formula (II) below:

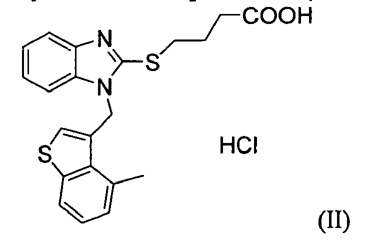

27. The crystal of the hydrochloride according to claim 26 showing a powder X-ray diffraction pattern with characteristic peaks at diffraction angles 2θ of approximately 7.0°, 7.8°, 22.7°, 24.1 °, and 26.2° (Crystal-HA).

28. The crystal of the hydrochloride according to claim 26 showing a powder X-ray diffraction pattern approximately the same as that shown in Figure 11 (Crystal-HA).

29. The crystal of the hydrochloride according to claim 26 showing a powder X-ray diffraction pattern with characteristic peaks at diffraction angles 2θ of approximately 8.5°, 11.9°, 19.7°, and 21.2° (Crystal-HB).

30. The crystal of the hydrochloride according to claim 26 showing a powder X-ray diffraction pattern approximately the same as that shown in Figure 12 (Crystal-HB).

31. The crystal of the hydrochloride according to claim 26 showing a powder X-ray diffraction pattern with characteristic peaks at diffraction angles 2θ of approximately 7.9°, 11.5°, 14.4°, and 16.8° (Crystal-HC).

32. The crystal of the hydrochloride according to claim 26 showing a powder X-ray diffraction pattern approximately the same as that shown in Figure 13 (Crystal-HC).

33. The crystal of the hydrochloride according to claim 26 whose infrared absorption spectrum in potassium bromide exhibits peaks at wavenumbers of approximately 1712, 1465, 1253, 1184, 1116, 872, and 752 cm⁻¹ (Crystal-HA).

34. The crystal of the hydrochloride according to claim 26 whose infrared absorption spectrum in potassium bromide exhibits the absorption pattern shown in Figure 14 (Crystal-HA).

35. The crystal of the hydrochloride according to claim 26 whose infrared absorption spectrum in potassium bromide exhibits peaks at wavenumbers of approximately 1708, 1458, 1242, 1103, 1028, 881, and 760 cm⁻¹ (Crystal-HB).

36. The crystal of the hydrochloride according to claim 26 whose infrared absorption spectrum in potassium bromide exhibits the absorption pattern shown in Figure 15 (Crystal-HB).

37. The crystal of the hydrochloride according to claim 26 whose infrared absorption spectrum in potassium bromide exhibits peaks at wavenumbers of approximately 1707, 1461, 1182, 1032, and 754 cm⁻¹ (Crystal-HC).

38. The crystal of the hydrochloride according to claim 26 whose infrared absorption spectrum in potassium bromide exhibits the absorption pattern shown in Figure 16 (Crystal-HC).

39. A crystal of 4-(1-((4-methylbenzothiophen-3-yl)methyl)benzimidazol-2-ylthio)butanoic acid methanesulfonate represented by the following formula (III):

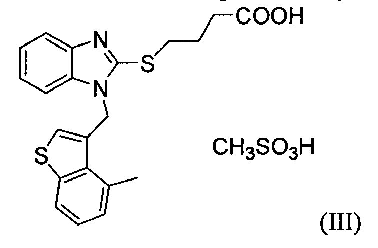

40. The crystal of the methanesulfonate according to claim 39 showing a powder X-ray diffraction pattern with characteristic peaks at diffraction angles 2θ of approximately 6.6°, 18.4°, and 21.3° (Crystal-MA).

41. The crystal of the methanesulfonate according to claim 39 showing a powder X-ray diffraction pattern approximately the same as that shown in Figure 17 (Crystal-MA).

42. The crystal of the methanesulfonate according to claim 39 showing a powder X-ray diffraction pattern with characteristic peaks at diffraction angles 2θ of approximately 8.3°, 9.6°, 13.7°, and 25.0° (Crystal-MB).

43. The crystal of the methanesulfonate according to claim 39 showing a powder X-ray diffraction pattern approximately the same as that shown in Figure 18 (Crystal-MB).

44. The crystal of the methanesulfonate according to claim 39 showing a powder X-ray diffraction pattern with characteristic peaks at diffraction angles 2θ of approximately 6.1°, 12.4°, 18.2°, 20.7°, and 24.2° (Crystal-MC).

45. The crystal of the methanesulfonate according to claim 39 showing a powder X-ray diffraction pattern approximately the same as that shown in Figure 19 (Crystal-MC).

46. The crystal of the methanesulfonate according to claim 39 showing a powder X-ray diffraction pattern with characteristic peaks at diffraction angles 2θ of approximately 9.1°, 12.6°, 14.7°, and 25.3° (Crystal-MD).

47. The crystal of the methanesulfonate according to claim 39 showing a powder X-ray diffraction pattern approximately the same as that shown in Figure 20 (Crystal-MD).

48. The crystal of the methanesulfonate according to claim 39 showing a powder X-ray diffraction pattern with characteristic peaks at diffraction angles 2θ of approximately 8.7°, 18.9°, and 22.6° (Crystal-ME).

49. The crystal of the methanesulfonate according to claim 39 showing a powder X-ray diffraction pattern approximately the same as that shown in Figure 21 (Crystal-ME).

50. The crystal of the methanesulfonate according to claim 39 whose infrared absorption spectrum in potassium bromide exhibits peaks at wavenumbers of approximately 1720, 1707, 1467, 1309, 1218, 1196, 1151, 1043, and 773 cm⁻¹ (Crystal-MA).

51. The crystal of the methanesulfonate according to claim 39 whose infrared absorption spectrum in potassium bromide exhibits the absorption pattern shown in Figure 22 (Crystal-MA).

52. The crystal of the methanesulfonate according to claim 39 whose infrared absorption spectrum in potassium bromide exhibits peaks at wavenumbers of approximately 1724, 1457, 1247, 1211, 1173, 1025, and 777 cm⁻¹ (Crystal-MB).

53. The crystal of the methanesulfonate according to claim 39 whose infrared absorption spectrum in potassium bromide exhibits the absorption pattern shown in Figure 23 (Crystal-MB).

54. The crystal of the methanesulfonate according to claim 39 whose infrared absorption spectrum in potassium bromide exhibits peaks at wavenumbers of approximately 1728, 1705, 1468, 1365, 1213, 1186, 1149, 1041, 881, and 773 cm⁻¹ (Crystal-MC).

55. The crystal of the methanesulfonate according to claim 39 whose infrared absorption spectrum in potassium bromide exhibits the absorption pattern shown in Figure 24 (Crystal-MC).

56. The crystal of the methanesulfonate according to claim 39 whose infrared absorption spectrum in potassium bromide exhibits peaks at wavenumbers of approximately 1716, 1461, 1240, 1136, 1041, 1041, and 764 cm⁻¹ (Crystal-MD).

57. The crystal of the methanesulfonate according to claim 39 whose infrared absorption spectrum in potassium bromide exhibits the absorption pattern shown in Figure 25(Crystal-MD).

58. The crystal of the methanesulfonate according to claim 39 whose infrared absorption spectrum in potassium bromide exhibits peaks at wavenumbers of approximately 1714, 1464, 1216, 1037, 874, and 771 cm⁻¹ (Crystal-ME).

59. The crystal of the methanesulfonate according to claim 39 whose infrared absorption spectrum in potassium bromide exhibits the absorption pattern shown in Figure 26 (Crystal-ME).

60. A pharmaceutical composition comprising the acid salt of 4-(1-((4-methylbenzothiophen-3-yl)methyl)benzimidazol-2-ylthio)butanoic acid or the crystal thereof according to claim 1 or 2 as an active ingredient.

61. The pharmaceutical composition according to claim 60 wherein the acid salt is sulfate, hydrochloride, or methanesulfonate.

62. A chymase inhibitory agent comprising the acid salt of 4-(1-((4-methylbenzothiophen-3-yl)methyl)benzimidazol-2-ylthio)butanoic acid or the crystal thereof according to claim 1 or 2 as an active ingredient.

63. The chymase inhibitory agent according to claim 62 wherein the acid salt is sulfate, hydrochloride, or methanesulfonate.

64. A preventive and/or therapeutic agent for inflammatory diseases, allergic diseases, respiratory diseases, circulatory diseases, or bone/cartilage metabolic diseases comprising the acid salt of 4-(1-((4-methylbenzothiophen-3-yl)methyl)benzimidazol-2-ylthio)butanoic acid or the crystal thereof according to claim 1 or 2 as an active ingredient.

65. The preventive and/or therapeutic agent according to claim 64 wherein the acid salt is sulfate, hydrochloride, or methanesulfonate.

66. A pharmaceutical composition comprising a crystal or a mixture of two or more forms of crystals selected from the crystals of the sulfate described in claims 5 to 25 as an active ingredient.

67. A chymase inhibitory agent comprising a crystal or a mixture of two or more forms of crystals selected from the crystals of the sulfate described in claims 5 to 25 as an active ingredient.

68. A preventive and/or therapeutic agent for inflammatory diseases, allergic diseases, respiratory diseases, circulatory diseases, or bone/cartilage metabolic diseases comprising a crystal or a mixture of two or more forms of crystals selected from the crystals of the sulfate described in claims 5 to 25 as an active ingredient.

69. A pharmaceutical composition comprising a crystal or a mixture of two or more forms of crystals selected from the crystals of the hydrochloride described in claims 26 to 38 as an active ingredient.

70. A chymase inhibitory agent comprising a crystal or a mixture of two or more forms of crystals selected from the crystals of the hydrochloride described in claims 26 to 38 as an active ingredient.

71. A preventive and/or therapeutic agent for inflammatory diseases, allergic diseases, respiratory diseases, circulatory diseases, or bone/cartilage metabolic diseases comprising a crystal or a mixture of two or more forms of crystals selected from the crystals of the hydrochloride described in claims 26 to 38 as an active ingredient.

72. A pharmaceutical composition comprising a crystal or a mixture of two or more forms of crystals selected from the crystals of the methanesulfonate described in claims 39 to 59 as an active ingredient.

73. A chymase inhibitory agent comprising a crystal or a mixture of two or more forms of crystals selected from the crystals of the methanesulfonate described in claims 39 to 59 as an active ingredient.

74. A preventive and/or therapeutic agent for inflammatory diseases, allergic diseases, respiratory diseases, circulatory diseases, or bone/cartilage metabolic diseases comprising a crystal or a mixture of two or more forms of crystals selected from the crystals of the methanesulfonate described in claims 39 to 59 as an active ingredient.

75. A pharmaceutical composition comprising a crystal or a mixture of two or more forms of crystals selected from the crystals described in claims 5 to 59 as an active ingredient.

76. A chymase inhibitory agent comprising a crystal, or a mixture of two or more forms of crystals, selected from the crystals described in claims 5 to 59 as an active ingredient.

77. A preventive and/or therapeutic agent for inflammatory diseases, allergic diseases, respiratory diseases, circulatory diseases, or bone/cartilage metabolic diseases comprising a crystal, or a mixture of two or more forms of crystals, selected from the crystals described in claims 5 to 59 as an active ingredient.
